# EUROPEAN PATENT SPECIFICATION

(11) **EP 3 124 034 B1**
(45) Date of publication and mention of the grant of the patent: **21.04.2021**
(21) Application number: 15768816.9
(22) Date of filing: 04.03.2015
(51) Int. Cl.: A61K 38/05, A61P 3/10, A61P 25/28, A61P 29/00, A61P 43/00, A61K 31/4172, A61K 8/64, A61Q 19/08, A61K 45/06, A61K 31/198, A23L 33/18, G01N 33/68

(54) **IMIDAZOLE DIPEPTIDE FOR TREATING DEMENTIA DUE TO AGING OR CEREBRAL ATROPHY**
IMIDAZOLDIPEPTID ZUR BEHANDLUNG VON ALTERSDEMENTIA ODER HIRNATROPHIE
DIPEPTIDE D'IMIDAZOLE POUR LE TRAITEMENT DE LA DEMENCE SENILE OU L'ATROPHIE CEREBRALE

(30) Priority: 28.03.2014 JP 2014069103; 11.07.2014 JP 2014142910
(43) Date of publication of application: 01.02.2017
(73) Proprietor: The University of Tokyo, Tokyo 113-8654 (JP); Kyushu University, National University Corporation, Fukuoka-shi, Fukuoka 812-8581 (JP); NH Foods Ltd., Osaka-shi, Osaka 530-0001 (JP); National Center of Neurology and Psychiatry, Kodaira-shi Tokyo 187-8551 (JP)
(72) Inventor: HISATSUNE Tatsuhiro, Tokyo 113-8654 (JP); TOTSUKA Mamoru, Tokyo 113-8654 (JP); SATSU Hideo, Tokyo 113-8654 (JP); KANEKO Jun, Tokyo 113-8654 (JP); KATAKURA Yoshinori, Fukuoka-shi Fukuoka 812-8581 (JP); SATO Mikako, Tsukuba-shi Ibaraki 300-2646 (JP); MATSUMOTO Takashi, Tsukuba-shi Ibaraki 300-2646 (JP); MORIMATSU Fumiki, Tsukuba-shi Ibaraki 300-2646 (JP); IMABAYASHI Etsuko, Kodaira-shi Tokyo 187-8551 (JP); MATSUDA Hiroshi, Kodaira-shi Tokyo 187-8551 (JP)
(74) Representative: Vossius & Partner Patentanwälte Rechtsanwälte mbB
(86) International application number: PCT/JP2015/056412
(87) International publication number: WO 2015/146522

(56) References cited:
- EP-A1- 0 710 485
- EP-A1- 1 285 654
- WO-A1-03/013514
- WO-A1-2007/116987
- KR-A- 20020 044 740
- US-A1- 2006 257 502
- US-B1- 6 335 361
- BRUNO HERCULANO ET AL.: 'beta-Alanyl-L-Histidine Rescues Cognitive Deficits Caused by Feeding a High Fat Diet in a Transgenic Mouse Model of Alzheimer's Disease' JOURNAL OF ALZHEIMER'S DISEASE vol. 33, no. 4, 2013, pages 983 - 997, XP055358545
- CARLO CORONA ET AL.: 'Effects of Dietary Supplementation of Carnosine on Mitochondrial Dysfunction, Amyloid Pathology, and Cognitive Deficits in 3xTg-AD Mice' PLOS ONE vol. 6, no. 3, March 2011, pages 1 - 8, XP055225492
- JAMES N.BARANIUK ET AL.: 'Carnosine Treatment for Gulf War Illness: A Randomized Controlled Trial' GLOBAL JOURNAL OF HEALTH SCIENCE vol. 5, no. 3, 2013, pages 69 - 81, XP055225493
- ALESSANDRA ALOISI ET AL.: 'Anti-Aggregating Effect of the Naturally Occurring Dipeptide Carnosine on Abeta1-42 Fibril Formation' PLOS ONE vol. 8, no. 7, July 2013, pages 1 - 12, XP055368672

### SEQUENCE LISTING

<110> The University of Tokyo Kyushu University, National University Corporation NH Foods Ltd. National Center of Neurology and Psychiatry
<120> Agent containing imidazoledipeptide
<130> 141979M
<150> JP 2014-069103
   <151> 2014-03-28
<150> JP 2014-142910
   <151> 2014-07-11
<160> 20
<170> PatentIn version 3.5
<210> 1
   <211> 1488
   <212> DNA
   <213> Homo sapiens
<400> 1
   atgccatgtg tgtggggtac gaccagtggg ccaccagcca gctcattggg accattttct      60
   tctgtgtggg aatcactact ttgctacaga caacgtttgg atgcagatgt ttcagttgcc     120
   aatggaacag cagagctgtt gcacacagaa cacatctggt atccccggat ccgagagatc     180
   cagggggcca tcatcatgtc ctcactgata gaagtagtca tcggcctcct cggcctgcct     240
   ggggctctac tgaagtacat cggtcccttg accattacac ccacggtggc cctaattggc     300
   ctctctggtt tccaggcagc gggggagaga gccgggaagc actggggcat tgccatgctg     360
   acaatattcc tagtattact gttttctcaa tacgccagaa atgttaaatt tcctctcccg     420
   atttataaat ccaagaaagg atggactgcg tacaagttac agctgttcaa aatgttccct     480
   atcatcctgg ccatcctggt atcctggctg ctctgcttca tcttcacggt gacagatgtc     540
   ttccctcccg acagcacaaa gtatggcttc tatgctcgca cagatgccag gcaaggcgtg     600
   cttctggtag ccccgtggtt taaggttcca tacccatttc agtggggact gcccaccgtg     660
   tctgcggccg gtgtcatcgg catgctcagt gccgtggtcg ccagcatcat cgagtctatt     720
   ggtgactact acgcctgtgc acggctgtcc tgtgccccac ccccccccat ccacgcaata     780
   aacaggggaa ttttcgtgga aggcctctcc tgtgttcttg atggcatatt tggtactggg     840
   aatggctcta cttcatccag tcccaacatt ggagttttgg gaattacaaa ggtcggcagc     900
   cgccgcgtga tacagtgcgg agcagccctc atgctcgctc tgggcatgat cgggaagttc     960
   agcgccctct ttgcgtccct tccggatcct gtgctgggag ccctgttctg cacgctcttt    1020
   ggaatgatca cagctgttgg cctctctaac ctgcagttca ttgatttaaa ttcttcccgg    1080
   aacctctttg tgcttggatt ttcgatcttc tttgggctcg tccttccaag ttacctcaga 1140
   cagaaccctc tggtcacagg gataacagga atcgatcaag tgttgaacgt ccttctcaca 1200
   actgctatgt ttgtaggggg ctgtgtggct tttatcctgg ataacaccat cccaggcact 1260
   ccagaggaaa gaggaatccg gaaatggaag aagggtgtgg gcaaagggaa caaatcactc 1320
   gacggcatgg agtcgtacaa tttgccattt ggcatgaaca ttataaaaaa atacagatgc 1380
   ttcagctact tacccatcag cccaaccttt gtgggctaca catggaaagg cctcaggaag 1440
   agcgacaaca gccggagttc agatgaagac tcccaggcca cgggatag 1488
<210> 2
   <211> 1710
   <212> DNA
   <213> Homo sapiens
<400> 2
   atggcgccca ccctggccac tgcccatcgg cgccgctggt ggatggcctg cacggccgtg 60
   ctggagaacc tcctcttctc ggcagtcctc ctgggctggg gctcgctgct catcatgctc 120
   aagtcagagg gcttttactc ctacctgtgt accgagccag agaatgtcac caatggcaca 180
   gtgggcggca cagcagagcc ggggcacgag gaggtgagct ggatgaacgg ctggctcagc 240
   tgccaggccc aggacgagat gctaaatttg gccttcactg tgggctcctt tctgctcagt 300
   gccatcaccc tgcccctggg tatcgtcatg gacaagtatg gcccgaggaa gctcaggctg 360
   ctgggcagcg cctgcttcgc ggtttcctgc ttgctgattg cgtacggagc aagtaaacca 420
   aacgctctct ccgtgctcat cttcatcgcc ctggctctga atggctttgg tgggatgtgt 480
   atgaccttca cctcattaac actgcccaac atgttcggcg accttcggtc cacgtttatt 540
   gccttgatga ttgggtccta cgcctcctcg gcagtcacct ttccaggaat caagctcatc 600
   tatgatgctg gtgtctcctt catcgtcgtc ctcgtggtct gggccggctg ctccgggctg 660
   gttttcctca actgcttctt taactggccc cttgagccct tcccggggcc ggaggacatg 720
   gactactcgg tgaagatcaa gttcagctgg ctgggctttg accacaagat cacagggaag 780
   cagttctaca agcaggtgac cacggtgggc cggcgcctga gtgtgggcag ctccatgagg 840
   agtgccaagg agcaggtggc gctgcaggag ggccacaagc tgtgcctgtc caccgtcgac 900
   ctggaggtga agtgccagcc ggatgccgca gtggccccct ccttcatgca cagcgtgttc 960
   agccccatcc tgctgctcag cctggtcacc atgtgcgtca cgcagctgcg gctcatcttc 1020
   tacatggggg ctatgaacaa catcctcaag ttcctggtca gcggcgacca gaagacagtt 1080
   ggcctctaca cctccatctt cggcgtgctc cagctgctgt gcctgctgac ggcccccgtc 1140
   attggctaca tcatggactg gaggctgaag gagtgtgaag acgcctccga ggagcccgag 1200
   gagaaagacg ccaaccaagg cgagaagaaa aagaagaagc gggaccggca gatccagaag 1260
   atcactaatg ccatgcgggc cttcgccttc accaacctgc tgctcgtggg ctttggggtg 1320
   acctgcctca ttcccaacct gcctctccag atcctctcct tcatcctgca cacaatcgtg 1380
   cgaggattca tccactccgc tgtcgggggc ctgtacgctg ccgtgtaccc ctccacccag 1440
   ttcggcagcc tcacgggact gcagtctctg atcagcgcgc tcttcgccct tctgcagcag 1500
   ccgctgtttc tggccatgat gggtcctctc cagggagacc ctctgtgggt gaacgtgggg 1560
   ctgctccttc tcagcctgct gggcttctgc ctcccgctct acctgatctg ctaccggcgc 1620
   cagctggagc ggcagctgca gcagaggcag gaggatgaca aactcttcct caaaatcaac 1680
   ggctcgtcca accaggaggc cttcgtgtag 1710
<210> 3
   <211> 1428
   <212> DNA
   <213> Homo sapiens
<400> 3
   atggccgttg tctcagagga cgactttcag cacagttcaa actccaccta cagaaccaca      60
   agcagcagtc tccgagctga ccaggaggca ctgcttgaga agctgctgga ccgcccgccc     120
   cctggcctgc agaggcccga ggaccgcttc tgtggcacat acatcatctt cttcagcctg     180
   ggcattggca gtctactgcc atggaacttc tttatcactg ccaaggagta ctggatgttc     240
   aaactccgca actcctccag cccagccacc ggggaggacc ctgagggctc agacatcctg     300
   aactactttg agagctacct tgccgttgcc tccaccgtgc cctccatgct gtgcctggtg     360
   gccaacttcc tgcttgtcaa cagggttgca gtccacatcc gtgtcctggc ctcactgacg     420
   gtcatcctgg ccatcttcat ggtgataact gcactggtga aggtggacac ttcctcctgg     480
   acccgtggct tttttgcggt caccattgtc tgcatggtga tcctcagcgg tgcctccact     540
   gtcttcagca gcagcatcta cggcatgacc ggctcctttc ctatgaggaa ctcccaggca     600
   ctgatatcag gaggagccat gggcgggacg gtcagcgccg tggcctcatt ggtggacttg     660
   gctgcatcca gtgatgtgag gaacagcgcc ctggccttct tcctgacggc cactgtcttc     720
   ctcgtgctct gcatgggact ctacctgctg ctgtccaggc tggagtatgc caggtactac     780
   atgaggcctg ttcttgcggc ccatgtgttt tctggtgaag aggagcttcc ccaggactcc     840
   ctcagtgccc cttcggtggc ctccagattc attgattccc acacaccccc tctccgcccc     900
   atcctgaaga agacggccag cctgggcttc tgtgtcacct acgtcttctt catcaccagc     960
   ctcatctacc ccgccatctg caccaacatc gagtccctca acaagggttc gggctcactg    1020
   tggaccacca agtttttcat ccccctcact accttcctcc tgtacaactt tgctgaccta    1080
   tgtggccggc agctcaccgc ctggatccag gtgccagggc ccaatagcaa ggcgctccca    1140
   gggttcgtgc tcctccggac ctgcctcatc cccctcttcg tgctctgtaa ctaccagccc    1200
   cgcgtccacc tgaagactgt ggtcttccag tccgatgtgt accccgcact cctcagctcc    1260
   ctgctggggc tcagcaacgg ctacctcagc accctggccc tcctctacgg gcctaagatt    1320
   gtgcccaggg agctggctga ggccacggga gtggtgatgt ccttttatgt gtgcttgggc    1380
   ttaacactgg gctcagcctg ctctaccctc ctggtgcacc tcatctag                 1428
<210> 4
   <211> 1095
   <212> DNA
   <213> Homo sapiens
<400> 4
   atgaataggg cccctctgaa gcggtccagg atcctgcaca tggcgctgac cggggcctca      60
   gacccctctg cagaggcaga ggccaacggg gagaagccct ttctgctgcg ggcattgcag     120
   atcgcgctgg tggtctccct ctactgggtc acctccatct ccatggtgtt ccttaataag     180
   tacctgctgg acagcccctc cctgcggctg gacaccccca tcttcgtcac cttctaccag     240
   tgcctggtga ccacgctgct gtgcaaaggc ctcagcgctc tggccgcctg ctgccctggt     300
   gccgtggact tccccagctt gcgcctggac ctcagggtgg cccgcagcgt cctgcccctg     360
   tcggtggtct tcatcggcat gatcaccttc aataacctct gcctcaagta cgtcggtgtg     420
   gccttctaca atgtgggccg ctcactcacc accgtcttca acgtgctgct ctcctacctg     480
   ctgctcaagc agaccacctc cttctatgcc ctgctcacct gcggtatcat catcgggggc     540
   ttctggcttg gtgtggacca ggagggggca gaaggcaccc tgtcgtggct gggcaccgtc     600
   ttcggcgtgc tggctagcct ctgtgtctcg ctcaacgcca tctacaccac gaaggtgctc     660
   ccggcggtgg acggcagcat ctggcgcctg actttctaca acaacgtcaa cgcctgcatc     720
   ctcttcctgc ccctgctcct gctgctcggg gagcttcagg ccctgcgtga ctttgcccag     780
   ctgggcagtg cccacttctg ggggatgatg acgctgggcg gcctgtttgg ctttgccatc     840
   ggctacgtga caggactgca gatcaagttc accagtccgc tgacccacaa tgtgtcgggc     900
   acggccaagg cctgtgccca gacagtgctg gccgtgctct actacgagga gaccaagagc     960
   ttcctctggt ggacgagcaa catgatggtg ctgggcggct cctccgccta cacctgggtc    1020
   aggggctggg agatgaagaa gactccggag gagcccagcc ccaaagacag cgagaagagc    1080
   gccatggggg tgtga                                                     1095
<210> 5
   <211> 966
   <212> DNA
   <213> Homo sapiens
<400> 5
   atggcgacgg gcggccagca gaaggagaac acgctgcttc acctcttcgc cggcgggtgt      60
   ggaggcacag ttggtgctat tttcacttgt ccactagaag tcattaagac acggttgcag     120
   tcttcaagat tagctctccg gacagtctac tatcctcagg ttcatctggg gaccattagt     180
   ggagctggaa tggtgagacc aacatccgtg acacctggac tctttcaggt tctgaagtcg     240
   atcttggaga aagagggacc aaagtcactt tttagaggct tgggtccaaa tttggttgga     300
   gttgcaccat caagggctgt atactttgca tgttactcca aagccaaaga gcaatttaat     360
   ggcattttcg tgcctaacag caatattgtg catattttct cagctggctc tgcagctttt     420
   atcacaaatt ccttaatgaa tcctatatgg atggttaaaa cccgaatgca gctagaacag     480
   aaagtgaggg gctctaagca gatgaataca ctccagtgtg ctcgttacgt ttaccagacc     540
   gaaggcattc gtggcttcta tagaggatta actgcctcgt atgctggaat ttccgaaact     600
   ataatctgct ttgctattta tgaaagttta aagaagtatc tgaaagaagc tccattagcc     660
   tcttctgcaa atgggactga gaaaaattcc acaagttttt ttggacttat ggcagctgct     720
   gctctttcta agggctgtgc ctcctgcatt gcttatccac acgaagtcat aaggacgagg     780
   ctccgggaag agggcaccaa gtacaagtct tttgtccaga cggcgcgcct ggtgttccgg     840
   gaagaaggct accttgcctt ttatagagga ctgtttgccc agcttatccg gcagatccca     900
   aatactgcca ttgtgttgtc tacttatgag ttaattgtgt acctgttaga agaccgtact     960
   cagtaa                                                                966
<210> 6
   <211> 2061
   <212> DNA
   <213> Homo sapiens
<400> 6
   atggccatag accggcggcg cgaggcggcg ggcggcgggc ctgggcggca gccggccccg      60
   gccgaggaga acggctccct gccgcccggg gacgcggcgg cctcggcgcc cctcggggga     120
   cgcgcgggcc ccggcggcgg cgcggagatc cagccgctgc ccccactgca tcctggaggc     180
   ggcccgcacc cgagctgctg ctccgcggct gcggccccga gcctcttgtt gctggactat     240
   gacgggtcgg tgctgccctt cctcgggggc ctgggcgggg gctatcagaa gaccctcgtg     300
   ctgctcacct ggatcccggc gctgttcatc ggcttcagcc agttctcgga ctcgttcctc     360
   ctggaccagc ccaacttctg gtgccgcggg gccggcaaag gcaccgagct ggcaggggtc     420
   accaccacag gccggggcgg ggacatgggc aactggacca gcctccccac cacccccttc     480
   gccactgccc cctgggaggc tgcgggcaac cggagcaaca gcagcggcgc ggacggaggc     540
   gacacaccac ccctgccatc ccctccggac aagggggaca acgcctccaa ctgtgactgc     600
   cgcgcatggg actacggcat ccgcgccggc ctcgtccaga acgtggtcag caagtgggat     660
   cttgtgtgtg ataatgcctg gaaggtccat atcgctaagt tctccttact ggttggatta     720
   atctttggct acctaataac tggatgcatt gctgactggg tcggccggcg gcctgtgctg     780
   ctgttttcca tcatcttcat tctgatcttt ggactgactg tggcactgtc agtgaatgtg 840
   acaatgttca gcacactcag gttctttgaa ggattttgcc tggctggaat cattctcacc 900
   ttgtatgctt tacgaataga gctgtgcccc cctggaaaac ggttcatgat tacgatggtg 960
   gcgagcttcg tggccatggc gggccagttc ctcatgcctg ggctagccgc cctgtgccgg 1020
   gattggcagg tgctgcaggc cctcatcatc tgccccttcc tgctcatgct gctctactgg 1080
   tcgatattcc ccgagtccct ccggtggcta atggccaccc agcagtttga gtctgcaaag 1140
   aggctgatcc tccacttcac acagaagaat cgcatgaacc ctgagggcga catcaagggt 1200
   gtgataccag agctggagaa agagctttcc cggaggccca agaaggtctg catcgtgaag 1260
   gtggtgggga cacggaacct gtggaagaac attgtggtcc tgtgtgtgaa ctcgctgacg 1320
   gggtacggga tccaccactg ctttgccagg agcatgatgg gccacgaggt gaaggtgccg 1380
   ctcctggaga acttctatgc tgactactat accacggcca gcatcgcgct ggtgtcctgc 1440
   ctggccatgt gcgtggtggt ccgattcctc gggcgcaggg gagggctgct gctcttcatg 1500
   atcctcaccg ccctggcctc actcctgcag ctcggcctcc tcaacctgat tggaaagtac 1560
   agccagcacc cagactcagg gatgagtgac agcgtcaagg acaaattttc catcgcgttt 1620
   tccatcgtgg gcatgtttgc ctcccatgcg gtggggagcc tcagcgtgtt cttctgtgcg 1680
   gagatcaccc cgacggtgat aaggtgtggc gggctggggc tggtgctggc cagcgcgggc 1740
   ttcggcatgc tgacggcacc catcatcgag ctgcacaacc agaaaggcta cttcctgcac 1800
   cacatcatct ttgcctgctg cacgctcatc tgcatcatct gcatcctcct gctgcccgag 1860
   agcagggacc agaacctgcc tgagaacatt tctaacgggg agcactacac gcgccagccg 1920
   ctgctgccgc acaagaaggg ggagcagcca ctgctgctca ccaacgccga gctcaaggac 1980
   tactcgggcc tccacgatgc cgcagccgcg ggtgacacac tgcccgaggg tgccacggcc 2040
   aacggcatga aggccatgta g 2061
<210> 7
   <211> 270
   <212> DNA
   <213> Homo sapiens
<400> 7
   atgaacgcca aggtcgtggt cgtgctggtc ctcgtgctga ccgcgctctg cctcagcgac 60
   gggaagcccg tcagcctgag ctacagatgc ccatgccgat tcttcgaaag ccatgttgcc 120
   agagccaacg tcaagcatct caaaattctc aacactccaa actgtgccct tcagattgta 180
   gcccggctga agaacaacaa cagacaagtg tgcattgacc cgaagctaaa gtggattcag 240
   gagtacctgg agaaagcttt aaacaagtaa 270
<210> 8
   <211> 285
   <212> DNA
   <213> Homo sapiens
<400> 8
   atggccccac tgaagatgct ggccctggtc accctcctcc tgggggcttc tctgcagcac      60
   atccacgcag ctcgagggac caatgtgggc cgggagtgct gcctggagta cttcaaggga     120
   gccattcccc ttagaaagct gaagacgtgg taccagacat ctgaggactg ctccagggat     180
   gccatcgttt ttgtaactgt gcagggcagg gccatctgtt cggaccccaa caacaagaga     240
   gtgaagaatg cagttaaata cctgcaaagc cttgagaggt cttga                     285
<210> 9
   <211> 510
   <212> DNA
   <213> Homo sapiens
<400> 9
   atggccccgc cctgggtgcc cgccatgggc ttcacgctgg cgcccagcct ggggtgcttc      60
   gtgggctccc gctttgtcca cggcgagggt ctccgctggt acgccggcct gcagaagccc     120
   tcgtggcacc cgccccactg ggtgctgggc cctgtctggg gcacgctcta ctcagccatg     180
   gggtacggct cctacctggt ctggaaagag ctgggaggct tcacagagaa ggctgtggtt     240
   cccctgggcc tctacactgg gcagctggcc ctgaactggg catggccccc catcttcttt     300
   ggtgcccgac aaatgggctg ggccttggtg gatctcctgc tggtcagtgg ggcggcggca     360
   gccactaccg tggcctggta ccaggtgagc ccgctggccg cccgcctgct ctacccctac     420
   ctggcctggc tggccttcac gaccacactc aactactgcg tatggcggga caaccatggc     480
   tggcgtgggg gacggcggct gccagagtga                                      510
<210> 10
   <211> 1122
   <212> DNA
   <213> Homo sapiens
<400> 10
   atgaccgagg tgctgtggcc ggctgtcccc aacgggacgg acgctgcctt cctggccggt      60
   ccgggttcgt cctgggggaa cagcacggtc gcctccactg ccgccgtctc ctcgtcgttc     120
   aaatgcgcct tgaccaagac gggcttccag ttttactacc tgccggctgt ctacatcttg     180
   gtattcatca tcggcttcct gggcaacagc gtggccatct ggatgttcgt cttccacatg     240
   aagccctgga gcggcatctc cgtgtacatg ttcaatttgg ctctggccga cttcttgtac     300
   gtgctgactc tgccagccct gatcttctac tacttcaata aaacagactg gatcttcggg     360
   gatgccatgt gtaaactgca gaggttcatc tttcatgtga acctctatgg cagcatcttg     420
   tttctgacat gcatcagtgc ccaccggtac agcggtgtgg tgtaccccct caagtccctg    480
   ggccggctca aaaagaagaa tgcgatctgt atcagcgtgc tggtgtggct cattgtggtg     540
   gtggcgatct cccccatcct cttctactca ggtaccgggg tccgcaaaaa caaaaccatc     600
   acctgttacg acaccacctc agacgagtac ctgcgaagtt atttcatcta cagcatgtgc     660
   acgaccgtgg ccatgttctg tgtccccttg gtgctgattc tgggctgtta cggattaatt     720
   gtgagagctt tgatttacaa agatctggac aactctcctc tgaggagaaa atcgatttac     780
   ctggtaatca ttgtactgac tgtttttgct gtgtcttaca tccctttcca tgtgatgaaa    840
   acgatgaact tgagggcccg gcttgatttt cagaccccag caatgtgtgc tttcaatgac     900
   agggtttatg ccacgtatca ggtgacaaga ggtctagcaa gtctcaacag ttgtgtggac     960
   cccattctct atttcttggc gggagatact ttcagaagga gactctcccg agccacaagg    1020
   aaagcttcta gaagaagtga ggcaaatttg caatccaaga gtgaagacat gaccctcaat    1080
   attttacctg agttcaagca gaatggagat acaagcctgt ga                       1122
<210> 11
   <211> 1113
   <212> DNA
   <213> Homo sapiens
<400> 11
   atgctggggg cagtggaagg ccccaggtgg aagcaggcgg aggacattag agacatctac 60
   gacttccgag atgttctggg cacgggggcc ttctcggagg tgatcctggc agaagataag 120
   aggacgcaga agctggtggc catcaaatgc attgccaagg aggccctgga gggcaaggaa 180
   ggcagcatgg agaatgagat tgctgtcctg cacaagatca agcaccccaa cattgtagcc 240
   ctggatgaca tctatgagag tgggggccac ctctacctca tcatgcagct ggtgtcgggt 300
   ggggagctct ttgaccgtat tgtggaaaaa ggcttctaca cggagcggga cgccagccgc 360
   ctcatcttcc aggtgctgga tgctgtgaaa tacctgcatg acctgggcat tgtacaccgg 420
   gatctcaagc cagagaatct gctgtactac agcctggatg aagactccaa aatcatgatc 480
   tccgactttg gcctctccaa gatggaggac ccgggcagtg tgctctccac cgcctgtgga 540
   actccgggat acgtggcccc tgaagtcctg gcccagaagc cctacagcaa ggctgtggat 600
   tgctggtcca taggtgtcat cgcctacatc ttgctctgcg gttaccctcc cttctatgac 660
   gagaatgatg ccaaactctt tgaacagatt ttgaaggccg agtacgagtt tgactctcct 720
   tactgggacg acatctctga ctctgccaaa gatttcatcc ggcacttgat ggagaaggac 780
   ccagagaaaa gattcacctg tgagcaggcc ttgcagcacc catggattgc aggagataca 840
   gctctagata agaatatcca ccagtcggtg agtgagcaga tcaagaagaa ctttgccaag 900
   agcaagtgga agcaagcctt caatgccacg gctgtggtgc ggcacatgag gaaactgcag 960
   ctgggcacca gccaggaggg gcaggggcag acggcgagcc atggggagct gctgacacca 1020
   gtggctgggg ggccggcagc tggctgttgc tgtcgagact gctgcgtgga gccgggcaca 1080
   gaactgtccc ccacactgcc ccaccagctc tag 1113
<210> 12
   <211> 2343
   <212> DNA
   <213> Homo sapiens
<400> 12
   atggcgccct acagcctact ggtgactcgg ctgcagaaag ctctgggtgt gcggcagtac      60
   catgtggcct cagtcctgtg ccaacgggcc aaggtggcga tgagccactt tgagcccaac     120
   gagtacatcc attatgacct gctagagaag aacattaaca ttgttcgcaa acgactgaac     180
   cggccgctga cactctcgga gaagattgtg tatggacacc tggatgaccc cgccagccag     240
   gaaattgagc gaggcaagtc gtacctgcgg ctgcggccgg accgtgtggc catgcaggat     300
   gcgacggccc agatggccat gctccagttc atcagcagcg ggctgtccaa ggtggctgtg     360
   ccatccacca tccactgtga ccatctgatt gaagcccagg ttgggggcga gaaagacctg     420
   cgccgggcca aggacatcaa ccaggaagtt tataatttcc tggcaactgc aggtgccaaa     480
   tatggcgtgg gcttctggaa gcctggatct ggaatcattc accagattat tctggaaaac     540
   tatgcgtacc ctggtgttct tctgattggc actgactccc acacccccaa tggtggcggc     600
   cttgggggca tctgcattgg agttgggggt gccgatgctg tggatgtcat ggctgggatc     660
   ccctgggagc tgaagtgccc caaggtgatt ggcgtgaagc tgacgggctc tctctccggt     720
   tggtcctcac ccaaagatgt gatcctgaag gtggcaggca tcctcacggt gaaaggtggc     780
   acaggtgcaa tcgtggaata ccacgggcct ggtgtagact ccatctcctg cactggcatg     840
   gcgacaatct gcaacatggg tgcagaaatt ggggccacca cttccgtgtt cccttacaac     900
   cacaggatga agaagtacct gagcaagacc ggccgggaag acattgccaa tctagctgat     960
   gaattcaagg atcacttggt gcctgaccct ggctgccatt atgaccaact aattgaaatt    1020
   aacctcagtg agctgaagcc acacatcaat gggcccttca cccctgacct ggctcaccct    1080
   gtggcagaag tgggcaaggt ggcagagaag gaaggatggc ctctggacat ccgagtgggt    1140
   ctaattggta gctgcaccaa ttcaagctat gaagatatgg ggcgctcagc agctgtggcc    1200
   aagcaggcac tggcccatgg cctcaagtgc aagtcccagt tcaccatcac tccaggttcc    1260
   gagcagatcc gcgccaccat tgagcgggac ggctatgcac agatcttgag ggatctgggt    1320
   ggcattgtcc tggccaatgc ttgtggcccc tgcattggcc agtgggacag gaaggacatc    1380
   aagaaggggg agaagaacac aatcgtcacc tcctacaaca ggaacttcac gggccgcaac    1440
   gacgcaaacc ccgagaccca tgcctttgtc acgtccccag agattgtcac agccctggcc    1500
   attgcgggaa ccctcaagtt caacccagag accgactacc tgacgggcac ggatggcaag    1560
   aagttcaggc tggaggctcc ggatgcagat gagcttccca aaggggagtt tgacccaggg 1620
   caggacacct accagcaccc acccaaggac agcagcgggc agcatgtgga cgtgagcccc 1680
   accagccagc gcctgcagct cctggagcct tttgacaagt gggatggcaa ggacctggag 1740
   gacctgcaga tcctcatcaa ggtcaaaggg aagtgtacca ctgaccacat ctcagctgct 1800
   ggcccctggc tcaagttccg tgggcacttg gataacatct ccaacaacct gctcattggt 1860
   gccatcaaca ttgaaaacgg caaggccaac tccgtgcgca atgccgtcac tcaggagttt 1920
   ggccccgtcc ctgacactgc ccgctactac aagaaacatg gcatcaggtg ggtggtgatc 1980
   ggagacgaga actacggcga gggctcgagc cgggagcatg cagctctgga gcctcgccac 2040
   cttgggggcc gggccatcat caccaagagc tttgccagga tccacgagac caacctgaag 2100
   aaacagggcc tgctgcctct gaccttcgct gacccggctg actacaacaa gattcaccct 2160
   gtggacaagc tgaccattca gggcctgaag gacttcaccc ctggcaagcc cctgaagtgc 2220
   atcatcaagc accccaacgg gacccaggag accatcctcc tgaaccacac cttcaacgag 2280
   acgcagattg agtggttccg cgctggcagt gccctcaaca gaatgaagga actgcaacag 2340
   tga 2343
<210> 13
   <211> 4503
   <212> DNA
   <213> Homo sapiens
<400> 13
   atggatccaa gtatgggtgt gaattctgtt accatttctg ttgagggtat gacttgcaat      60
   tcctgtgttt ggaccattga gcagcagatt ggaaaagtga atggtgtgca tcacattaag     120
   gtatcactgg aagaaaaaaa tgcaactatt atttatgacc ctaaactaca gactccaaag     180
   accctacagg aagctattga tgacatgggc tttgatgctg ttatccataa tcctgaccct     240
   ctccctgttt taactgacac cttgtttctg actgttacgg cgtcactgac tttgccatgg     300
   gaccatatcc aaagcacatt gctgaagacc aagggtgtga cagacattaa aatttaccct     360
   cagaaaagaa ctgtagcagt gacaataatc ccttctatag tgaatgccaa tcagataaaa     420
   gagctggttc cagaactcag tttagatact gggacactgg agaaaaagtc aggagcttgt     480
   gaagatcata gtatggctca agctggtgaa gtcgtgctga agatgaaagt ggaagggatg     540
   acctgccatt catgtactag cactattgaa ggaaaaattg ggaaactgca aggtgttcag     600
   cgaattaaag tctccctgga caatcaagaa gctactattg tttatcaacc tcatcttatc     660
   tcagtagagg aaatgaaaaa gcagattgaa gctatgggct ttccagcatt tgtcaaaaag     720
   cagcccaagt acctcaaatt gggagctatt gatgtagaac gtctaaagaa cacaccagtt     780
   aaatcctcag aagggtcaca gcaaaggagt ccatcatata ccaatgattc aacagccact     840
   ttcatcattg atggcatgca ttgtaaatca tgtgtgtcaa atattgaaag tactttatct 900
   gcactccaat atgtaagcag catagtagtt tctttagaga ataggtctgc cattgtgaag 960
   tataatgcaa gctcagtcac tccagaatcc ctgagaaaag caatagaggc tgtatcaccg 1020
   gggctatata gagttagtat cacaagtgaa gttgagagta cctcaaactc tccctccagc 1080
   tcatctcttc agaagattcc tttgaatgta gttagccagc ctctgacaca agaaactgtg 1140
   ataaacattg atggcatgac ttgtaattcc tgtgtgcagt ctattgaggg tgtcatatca 1200
   aaaaagccag gtgtaaaatc catacgagtc tcccttgcaa atagcaatgg gactgttgag 1260
   tatgatcctc tactaacctc tccagaaacg ttgagaggag caatagaaga catgggattt 1320
   gatgctacct tgtcagacac gaatgagccg ttggtagtaa tagctcagcc ttcatcggaa 1380
   atgccgcttt tgacttcaac taatgaattt tatactaaag ggatgacacc agttcaagac 1440
   aaggaggaag gaaagaattc atctaagtgt tacatacagg tcactggcat gacttgcgct 1500
   tcctgtgtag caaacattga acggaattta aggcgggaag aaggaatata ttctatactt 1560
   gtggccctga tggctggcaa ggcagaagta aggtataatc ctgctgttat acaaccccca 1620
   atgatagcag agttcatccg agaacttgga tttggagcca ctgtgataga aaatgctgat 1680
   gaaggagatg gtgttttgga acttgttgtg aggggaatga cgtgtgcctc ctgcgtacat 1740
   aaaatagagt ctagtctcac aaaacacaga gggatcctat actgctccgt ggccctggca 1800
   accaacaaag cacatattaa atatgaccca gaaattattg gtcctagaga tattatccat 1860
   acaattgaaa gcttaggttt tgaagcttct ttggtcaaga aggatcggtc agcaagtcac 1920
   ttagatcata aacgagaaat aagacaatgg agacggtctt ttcttgtgag tctgtttttc 1980
   tgtattcctg taatggggct gatgatatat atgatggtta tggaccacca ctttgcaact 2040
   cttcaccata atcaaaacat gagtaaagaa gaaatgatca accttcattc ttctatgttc 2100
   ctggagcgcc agattcttcc aggattgtct gttatgaatt tgctgtcctt tttattgtgt 2160
   gtacctgtac agtttttcgg aggctggtac ttctacattc aggcttataa agcactgaag 2220
   cataagacag caaatatgga cgtactgatt gtgctggcaa ccaccattgc atttgcctac 2280
   tctttgatta ttcttctagt tgcaatgtat gagagagcca aagtgaaccc tattactttc 2340
   tttgacacac cccctatgct gtttgtgttt attgcactag gccgatggct ggaacatata 2400
   gcaaagggca aaacatcaga ggctcttgca aagttaattt cactacaagc tacagaagca 2460
   actattgtaa ctcttgattc tgataatatc ctcctcagtg aagaacaagt ggatgtggaa 2520
   cttgtacaac gtggagatat cattaaagta gttccaggag gcaaatttcc agtggatggt 2580
   cgtgttattg aaggacattc tatggtagat gagtccctca tcacagggga ggcaatgcct 2640
   gtggctaaga aacctggcag cacagtgatt gctggttcca ttaaccagaa cgggtcactg 2700
   cttatctgcg caacacatgt tggagcagac acaacccttt ctcaaattgt caaacttgtg 2760
   gaagaggcac aaacatcaaa ggctcctatc cagcagtttg cagacaaact cagtggctat 2820
   tttgttcctt ttattgtttt tgtttccatt gccaccctct tggtatggat tgtaattgga 2880
   tttctgaatt ttgaaattgt ggaaacctac tttcctggct acaatagaag tatctcccga 2940
   acagaaacga taatacgatt tgctttccaa gcctctatca cagttctgtg tattgcatgt 3000
   ccctgttcac tgggactggc cactccaact gctgtgatgg tgggtacagg agtaggtgct 3060
   caaaatggca tactaataaa aggtggagag ccattggaga tggctcataa ggtaaaggta 3120
   gtggtatttg ataagactgg aaccattact cacggaaccc cagtggtgaa tcaagtaaag 3180
   gttctaactg aaagtaacag aatatcacac cataaaatct tggccattgt gggaactgct 3240
   gaaagtaaca gtgaacaccc tctaggaaca gccataacca aatattgcaa acaggagctg 3300
   gacactgaaa ccttgggtac ctgcatagat ttccaggttg tgccaggctg tggtattagc 3360
   tgtaaagtca ccaatattga aggcttgcta cataagaata actggaatat agaggacaat 3420
   aatattaaaa atgcatccct ggttcaaatt gatgccagta atgaacagtc atcaacttcg 3480
   tcttccatga ttattgatgc ccagatctca aatgctctta atgctcagca gtataaagtc 3540
   ctcattggta accgggagtg gatgattaga aatggtcttg tcattaataa cgatgtaaat 3600
   gatttcatga ctgaacatga gagaaaaggt cggactgctg tattagtagc agttgatgat 3660
   gagctgtgtg gcttgatagc cattgcagac acagtgaagc ctgaagcaga actggctatc 3720
   catattctga aatctatggg cttagaagta gttctgatga ctggagacaa cagtaaaaca 3780
   gctagatcta ttgcttctca ggttggcatt actaaggtgt ttgctgaagt tctaccttct 3840
   cacaaggttg ctaaagtgaa gcaacttcaa gaggagggga aacgggtagc aatggtggga 3900
   gatggaatca atgactcccc agctctggca atggctaatg tgggaattgc tattggcaca 3960
   ggcacagatg tagccattga agcagctgat gtggttttga taaggaatga tcttctggat 4020
   gtagtggcaa gtattgactt atcaagaaag acagtcaaga ggattcggat aaattttgtc 4080
   tttgctctaa tttataatct ggttggaatt cccatagctg ctggagtttt tatgcccatt 4140
   ggtttggttt tgcagccctg gatgggatct gcagcaatgg ctgcttcatc tgtttctgta 4200
   gtactttctt ctctcttcct taaactttac aggaaaccaa cttacgagag ttatgaactg 4260
   cctgcccgga gccagatagg acagaagagt ccttcagaaa tcagcgttca tgttggaata 4320
   gatgatacct caaggaattc tcctaaactg ggtttgctgg accggattgt taattatagc 4380
   agagcctcta taaactcact actgtctgat aaacgctccc taaacagtgt tgttaccagt 4440
   gaacctgaca agcactcact cctggtggga gacttcaggg aagatgatga cactgcatta 4500
   taa 4503
<210> 14
   <211> 3720
   <212> DNA
   <213> Homo sapiens
<400> 14
   atgagccgcc tgctctggag gaaggtggcc ggcgccaccg tcgggccagg gccggttcca 60
   gctccggggc gctgggtctc cagctccgtc cccgcgtccg accccagcga cgggcagcgg 120
   cggcggcagc agcagcagca gcagcagcag cagcagcaac agcagcctca gcagccgcaa 180
   gtgctatcct cggagggcgg gcagctgcgg cacaacccat tggacatcca gatgctctcg 240
   agagggctgc acgagcaaat cttcgggcaa ggaggggaga tgcctggcga ggccgcggtg 300
   cgccgcagcg tcgagcacct gcagaagcac gggctctggg ggcagccagc cgtgcccttg 360
   cccgacgtgg agctgcgcct gccgcccctc tacggggaca acctggacca gcacttccgc 420
   ctcctggccc agaagcagag cctgccctac ctggaggcgg ccaacttgct gttgcaggcc 480
   cagctgcccc cgaagccccc ggcttgggcc tgggcggagg gctggacccg gtacggcccc 540
   gagggggagg ccgtacccgt ggccatcccc gaggagcggg ccctggtgtt cgacgtggag 600
   gtctgcttgg cagagggaac ttgccccaca ttggcggtgg ccatatcccc ctcggcctgg 660
   tattcctggt gcagccagcg gctggtggaa gagcgttact cttggaccag ccagctgtcg 720
   ccggctgacc tcatccccct ggaggtccct actggtgcca gcagccccac ccagagagac 780
   tggcaggagc agttagtggt ggggcacaat gtttcctttg accgagctca tatcagggag 840
   cagtacctga tccagggttc ccgcatgcgt ttcctggaca ccatgagcat gcacatggcc 900
   atctcagggc taagcagctt ccagcgcagt ctgtggatag cagccaagca gggcaaacac 960
   aaggtccagc cccccacaaa gcaaggccag aagtcccaga ggaaagccag aagaggccca 1020
   gcgatctcat cctgggactg gctggacatc agcagtgtca acagtctggc agaggtgcac 1080
   agactttatg taggggggcc tcccttagag aaggagcctc gagaactgtt tgtgaagggc 1140
   accatgaagg acattcgtga gaacttccag gacctgatgc agtactgtgc ccaggacgtg 1200
   tgggccaccc atgaggtttt ccagcagcag ctaccgctct tcttggagag gtgtccccac 1260
   ccagtgactc tggccggcat gctggagatg ggtgtctcct acctgcctgt caaccagaac 1320
   tgggagcgtt acctggcaga ggcacagggc acttatgagg agctccagcg ggagatgaag 1380
   aagtcgttga tggatctggc caatgatgcc tgccagctgc tctcaggaga gaggtacaaa 1440
   gaagacccct ggctctggga cctggagtgg gacctgcaag aatttaagca gaagaaagct 1500
   aagaaggtga agaaggaacc agccacagcc agcaagttgc ccatcgaggg ggctggggcc 1560
   cctggtgatc ccatggatca ggaagacctc ggcccctgca gtgaggagga ggagtttcaa 1620
   caagatgtca tggcccgcgc ctgcttgcag aagctgaagg ggaccacaga gctcctgccc 1680
   aagcggcccc agcaccttcc tggacaccct ggatggtacc ggaagctctg cccccggcta 1740
   gacgaccctg catggacccc gggccccagc ctcctcagcc tgcagatgcg ggtcacacct 1800
   aaactcatgg cacttacctg ggatggcttc cctctgcact actcagagcg tcatggctgg 1860
   ggctacttgg tgcctgggcg gcgggacaac ctggccaagc tgccgacagg taccaccctg 1920
   gagtcagctg gggtggtctg cccctacaga gccatcgagt ccctgtacag gaagcactgt 1980
   ctcgaacagg ggaagcagca gctgatgccc caggaggccg gcctggcgga ggagttcctg 2040
   ctcactgaca atagtgccat atggcaaacg gtagaagaac tggattactt agaagtggag 2100
   gctgaggcca agatggagaa cttgcgagct gcagtgccag gtcaacccct agctctgact 2160
   gcccgtggtg gccccaagga cacccagccc agctatcacc atggcaatgg accttacaac 2220
   gacgtggaca tccctggctg ctggtttttc aagctgcctc acaaggatgg taatagctgt 2280
   aatgtgggaa gcccctttgc caaggacttc ctgcccaaga tggaggatgg caccctgcag 2340
   gctggcccag gaggtgccag tgggccccgt gctctggaaa tcaacaaaat gatttctttc 2400
   tggaggaacg cccataaacg tatcagctcc cagatggtgg tgtggctgcc caggtcagct 2460
   ctgccccgtg ctgtgatcag gcaccccgac tatgatgagg aaggcctcta tggggccatc 2520
   ctgccccaag tggtgactgc cggcaccatc actcgccggg ctgtggagcc cacatggctc 2580
   accgccagca atgcccggcc tgaccgagta ggcagtgagt tgaaagccat ggtgcaggcc 2640
   ccacctggct acacccttgt gggtgctgat gtggactccc aagagctgtg gattgcagct 2700
   gtgcttggag acgcccactt tgccggcatg catggctgca cagcctttgg gtggatgaca 2760
   ctgcagggca ggaagagcag gggcactgat ctacacagta agacagccac tactgtgggc 2820
   atcagccgtg agcatgccaa aatcttcaac tacggccgca tctatggtgc tgggcagccc 2880
   tttgctgagc gcttactaat gcagtttaac caccggctca cacagcagga ggcagctgag 2940
   aaggcccagc agatgtacgc tgccaccaag ggcctccgct ggtatcggct gtcggatgag 3000
   ggcgagtggc tggtgaggga gttgaacctc ccagtggaca ggactgaggg tggctggatt 3060
   tccctgcagg atctgcgcaa ggtccagaga gaaactgcaa ggaagtcaca gtggaagaag 3120
   tgggaggtgg ttgctgaacg ggcatggaag gggggcacag agtcagaaat gttcaataag 3180
   cttgagagca ttgctacgtc tgacatacca cgtaccccgg tgctgggctg ctgcatcagc 3240
   cgagccctgg agccctcggc tgtccaggaa gagtttatga ccagccgtgt gaattgggtg 3300
   gtacagagct ctgctgttga ctacttacac ctcatgcttg tggccatgaa gtggctgttt 3360
   gaagagtttg ccatagatgg gcgcttctgc atcagcatcc atgacgaggt tcgctacctg 3420
   gtgcgggagg aggaccgcta ccgcgctgcc ctggccttgc agatcaccaa cctcttgacc 3480
   aggtgcatgt ttgcctacaa gctgggtctg aatgacttgc cccagtcagt cgcctttttc 3540
   agtgcagtcg atattgaccg gtgcctcagg aaggaagtga ccatggattg taaaacccct 3600
   tccaacccaa ctgggatgga aaggagatac gggattcccc agggtgaagc gctggatatt 3660
   taccagataa ttgaactcac caaaggctcc ttggaaaaac gaagccagcc tggaccatag 3720
<210> 15
   <211> 1182
   <212> DNA
   <213> Homo sapiens
<400> 15
   atggcgctga aggtagcgac cgtcgccggc agcgccgcga aggcggtgct cgggccagcc 60
   cttctctgcc gtccctggga ggttctaggc gcccacgagg tcccctcgag gaacatcttt 120
   tcagaacaaa caattcctcc gtccgctaag tatggcgggc ggcacacggt gaccatgatc 180
   ccaggggatg gcatcgggcc agagctcatg ctgcatgtca agtccgtctt caggcacgca 240
   tgtgtaccag tggactttga agaggtgcac gtgagttcca atgctgatga agaggacatt 300
   cgcaatgcca tcatggccat ccgccggaac cgcgtggccc tgaagggcaa catcgaaacc 360
   aaccataacc tgccaccgtc gcacaaatct cgaaacaaca tccttcgcac cagcctggac 420
   ctctatgcca acgtcatcca ctgtaagagc cttccaggcg tggtgacccg gcacaaggac 480
   atagacatcc tcattgtccg ggagaacaca gagggcgagt acagcagcct ggagcatgag 540
   agtgtggcgg gagtggtgga gagcctgaag atcatcacca aggccaagtc cctgcgcatt 600
   gccgagtatg ccttcaagct ggcgcaggag agcgggcgca agaaagtgac ggccgtgcac 660
   aaggccaaca tcatgaaact gggcgatggg cttttcctcc agtgctgcag ggaggtggca 720
   gcccgctacc ctcagatcac cttcgagaac atgattgtgg ataacaccac catgcagctg 780
   gtgtcccggc cccagcagtt tgatgtcatg gtgatgccca atctctatgg caacatcgtc 840
   aacaatgtct gcgcgggact ggtcgggggc ccaggccttg tggctggggc caactatggc 900
   catgtgtacg cggtgtttga aacagctacg aggaacaccg gcaagagtat cgccaataag 960
   aacatcgcca accccacggc caccctgctg gccagctgca tgatgctgga ccacctcaag 1020
   ctgcactcct atgccacctc catccgtaag gctgtcctgg catccatgga caatgagaat 1080
   atgcacactc cggacatcgg gggccagggc acaacatctg aagccatcca ggacgtcatc 1140
   cgccacatcc gcgtcatcaa cggccgggcc gtggaggcct ag 1182
<210> 16
   <211> 930
   <212> DNA
   <213> Homo sapiens
<400> 16
   atggttgggt tcaaggccac agatgtgccc cctactgcca ctgtgaagtt tcttggggct 60
   ggcacagctg cctgcatcgc agatctcatc acctttcctc tggatactgc taaagtccgg 120
   ttacagatcc aaggagaaag tcaggggcca gtgcgcgcta cagccagcgc ccagtaccgc 180
   ggtgtgatgg gcaccattct gaccatggtg cgtactgagg gcccccgaag cctctacaat 240
   gggctggttg ccggcctgca gcgccaaatg agctttgcct ctgtccgcat cggcctgtat 300
   gattctgtca aacagttcta caccaagggc tctgagcatg ccagcattgg gagccgcctc 360
   ctagcaggca gcaccacagg tgccctggct gtggctgtgg cccagcccac ggatgtggta 420
   aaggtccgat tccaagctca ggcccgggct ggaggtggtc ggagatacca aagcaccgtc 480
   aatgcctaca agaccattgc ccgagaggaa gggttccggg gcctctggaa agggacctct 540
   cccaatgttg ctcgtaatgc cattgtcaac tgtgctgagc tggtgaccta tgacctcatc 600
   aaggatgccc tcctgaaagc caacctcatg acagatgacc tcccttgcca cttcacttct 660
   gcctttgggg caggcttctg caccactgtc atcgcctccc ctgtagacgt ggtcaagacg 720
   agatacatga actctgccct gggccagtac agtagcgctg gccactgtgc ccttaccatg 780
   ctccagaagg aggggccccg agccttctac aaagggttca tgccctcctt tctccgcttg 840
   ggttcctgga acgtggtgat gttcgtcacc tatgagcagc tgaaacgagc cctcatggct 900
   gcctgcactt cccgagaggc tcccttctga 930
<210> 17
   <211> 1338
   <212> DNA
   <213> Homo sapiens
<400> 17
   atggcggtag cgatcgctgc agcgagggtc tggcggctaa accgtggttt gagccaggct 60
   gccctcctgc tgctgcggca gcctggggct cggggactgg ctagatctca cccccccagg 120
   cagcagcagc agttttcatc tctggatgac aagccccagt tcccaggggc ctcggcggag 180
   tttatagata agttggaatt catccagccc aacgtcatct ctggaatccc catctaccgc 240
   gtcatggacc ggcaaggcca gatcatcaac cccagcgagg acccccacct gccgaaggag 300
   aaggtgctga agctctacaa gagcatgaca ctgcttaaca ccatggaccg catcctctat 360
   gagtctcagc ggcagggccg gatctccttc tacatgacca actatggtga ggagggcacg 420
   cacgtgggga gtgccgccgc cctggacaac acggacctgg tgtttggcca gtaccgggag 480
   gcaggtgtgc tgatgtatcg ggactacccc ctggaactat tcatggccca gtgctatggc 540
   aacatcagtg acttgggcaa ggggcgccag atgcctgtcc actacggctg caaggaacgc 600
   cacttcgtca ctatctcctc tccactggcc acgcagatcc ctcaggcggt gggggcggcg 660
   tacgcagcca agcgggccaa tgccaacagg gtcgtcatct gttacttcgg cgagggggca 720
   gccagtgagg gggacgccca tgccggcttc aacttcgctg ccacacttga gtgccccatc 780
   atcttcttct gccggaacaa tggctacgcc atctccacgc ccacctctga gcagtatcgc 840
   ggcgatggca ttgcagcacg aggccccggg tatggcatca tgtcaatccg cgtggatggt 900
   aatgatgtgt ttgccgtata caacgccaca aaggaggccc gacggcgggc tgtggcagag 960
   aaccagccct tcctcatcga ggccatgacc tacaggatcg ggcaccacag caccagtgac 1020
   gacagttcag cgtaccgctc ggtggatgag gtcaattact gggataaaca ggaccacccc 1080
   atctcccggc tgcggcacta tctgctgagc caaggctggt gggatgagga gcaggagaag 1140
   gcctggagga agcagtcccg caggaaggtg atggaggcct ttgagcaggc cgagcggaag 1200
   cccaaaccca accccaacct actcttctca gacgtgtatc aggagatgcc cgcccagctc 1260
   cgcaagcagc aggagtctct ggcccgccac ctgcagacct acggggagca ctacccactg 1320
   gatcacttcg ataagtga 1338
<210> 18
   <211> 1962
   <212> DNA
   <213> Homo sapiens
<400> 18
   atgcggctcc ctgacctgag accctggacc tccctgctgc tggtggacgc ggctttactg      60
   tggctgcttc agggccctct ggggactttg cttcctcaag ggctgccagg actatggctg     120
   gaggggaccc tgcggctggg agggctgtgg gggctgctaa agctaagagg gctgctggga     180
   tttgtgggga cactgctgct cccgctctgt ctggccaccc ccctgactgt ctccctgaga     240
   gccctggtcg cgggggcctc acgtgctccc ccagccagag tcgcttcagc cccttggagc     300
   tggctgctgg tggggtacgg ggctgcgggg ctcagctggt cactgtgggc tgttctgagc     360
   cctcctggag cccaggagaa ggagcaggac caggtgaaca acaaagtctt gatgtggagg     420
   ctgctgaagc tctccaggcc ggacctgcct ctcctcgttg ccgccttctt cttccttgtc     480
   cttgctgttt tgggtgagac attaatccct cactattctg gtcgtgtgat tgacatcctg     540
   ggaggtgatt ttgaccccca tgcctttgcc agtgccatct tcttcatgtg cctcttctcc     600
   tttggcagct cactgtctgc aggctgccga ggaggctgct tcacctacac catgtctcga     660
   atcaacttgc ggatccggga gcagcttttc tcctccctgc tgcgccagga cctcggtttc     720
   ttccaggaga ctaagacagg ggagctgaac tcacggctga gctcggatac caccctgatg     780
   agtaactggc ttcctttaaa tgccaatgtg ctcttgcgaa gcctggtgaa agtggtgggg     840
   ctgtatggct tcatgctcag catatcgcct cgactcaccc tcctttctct gctgcacatg     900
   cccttcacaa tagcagcgga gaaggtgtac aacacccgcc atcaggaagt gcttcgggag     960
   atccaggatg cagtggccag ggcggggcag gtggtgcggg aagccgttgg agggctgcag    1020
   accgttcgca gttttggggc cgaggagcat gaagtctgtc gctataaaga ggcccttgaa    1080
   caatgtcggc agctgtattg gcggagagac ctggaacgcg ccttgtacct gctcgtaagg    1140
   agggtgctgc acttgggggt gcagatgctg atgctgagct gtgggctgca gcagatgcag    1200
   gatggggagc tcacccaggg cagcctgctt tcctttatga tctaccagga gagcgtgggg    1260
   agctatgtgc agaccctggt atacatatat ggggatatgc tcagcaacgt gggagctgca    1320
   gagaaggttt tctcctacat ggaccgacag ccaaatctgc cttcacctgg cacgcttgcc    1380
   cccaccactc tgcagggggt tgtgaaattc caagacgtct cctttgcata tcccaatcgc    1440
   cctgacaggc ctgtgctcaa ggggctgacg tttaccctac gtcctggtga ggtgacggcg    1500
   ctggtgggac ccaatgggtc tgggaagagc acagtggctg ccctgctgca gaatctgtac    1560
   cagcccacag ggggacaggt gctgctggat gaaaagccca tctcacagta tgaacactgc     1620
   tacctgcaca gccaggtggt ttcagttggg caggagcctg tgctgttctc cggttctgtg    1680
   aggaacaaca ttgcttatgg gctgcagagc tgcgaagatg ataaggtgat ggcggctgcc    1740
   caggctgccc acgcagatga cttcatccag gaaatggagc atggaatata cacagatgta    1800
   ggggagaagg gaagccagct ggctgcggga cagaaacaac gtctggccat tgcccgggcc    1860
   cttgtacgag acccgcgggt cctcatcctg gatgaggcta ctagtgccct agatgtgcag    1920
   tgcgagcagg ccaaaaccct ttggaagttc atgatatttt ga                       1962
<210> 19
   <211> 1425
   <212> DNA
   <213> Homo sapiens
<400> 19
   atggcggccc gggcgggttt ccagtctgtg gctccaagcg gcggcgccgg agcctcagga 60
   ggggcgggcg cggctgctgc cttgggcccg ggcggaactc cggggcctcc tgtgcgaatg 120
   ggcccggctc cgggtcaagg gctgtaccgc tccccgatgc ccggagcggc ctatccgaga 180
   ccaggtatgt tgccaggcag ccgaatgaca cctcagggac cttccatggg accccctggc 240
   tatgggggga acccttcagt ccgacctggc ctggcccagt cagggatgga tcagtcccgc 300
   aagagacctg cccctcagca gatccagcag gtccagcagc aggcggtcca aaatcgaaac 360
   cacaatgcaa agaaaaagaa gatggctgac aaaattctac ctcaaaggat tcgtgaactg 420
   gtaccagaat cccaggccta tatggatctc ttggcttttg aaaggaaact ggaccagact 480
   atcatgagga aacggctaga tatccaagag gccttgaaac gtcccatcaa gcaaaaacgg 540
   aagctgcgaa ttttcatttc taacactttc aatccggcta agtcagatgc cgaggatggg 600
   gaagggacgg tggcttcctg ggagcttcgg gtagaaggac ggctcctgga ggattcagcc 660
   ttgtccaaat atgatgccac taaacaaaag aggaagttct cttccttttt taagtccttg 720
   gtgattgaac tggacaaaga cctgtatggg ccagacaacc atctggtaga atggcacagg 780
   accgccacta cccaggagac cgatggcttt caggtgaagc ggccgggaga cgtgaatgta 840
   cggtgtactg tcctactgat gctggattac cagcctcccc agtttaaatt agacccccgc 900
   ctagctcgac tcctgggcat ccatacccag actcgtccag tgatcatcca agcactgtgg 960
   caatatatta agacacataa gctccaggac cctcacgagc gggagtttgt catctgtgac 1020
   aagtacctgc agcagatctt tgagtctcaa cgtatgaagt tttcagagat ccctcagcgg 1080
   ctccatgcct tgcttatgcc accagaacct atcatcatta atcatgtcat cagtgttgac 1140
   ccgaatgatc agaaaaagac agcttgttat gacattgatg ttgaagtgga tgacaccttg 1200
   aagacccaga tgaattcttt tctgctgtcc actgccagcc aacaggagat tgctactcta 1260
   gacaacaaga caatgactga tgtggtgggt aacccagagg aggagcgccg agctgagttc 1320
   tacttccagc cctgggctca ggaggctgtg tgccgatact tctactccaa ggtgcagcag 1380
   agacgacaag aattagagca agccctggga atccggaata catag 1425
<210> 20
   <211> 1068
   <212> DNA
   <213> Homo sapiens (SIRT6)
<400> 20
   atgtcggtga attacgcggc ggggctgtcg ccgtacgcgg acaagggcaa gtgcggcctc      60
   ccggagatct tcgacccccc ggaggagctg gagcggaagg tgtgggaact ggcgaggctg     120
   gtctggcagt cttccagtgt ggtgttccac acgggtgccg gcatcagcac tgcctctggc     180
   atccccgact tcaggggtcc ccacggagtc tggaccatgg aggagcgagg tctggccccc     240
   aagttcgaca ccacctttga gagcgcgcgg cccacgcaga cccacatggc gctggtgcag     300
   ctggagcgcg tgggcctcct ccgcttcctg gtcagccaga acgtggacgg gctccatgtg     360
   cgctcaggct tccccaggga caaactggca gagctccacg ggaacatgtt tgtggaagaa     420
   tgtgccaagt gtaagacgca gtacgtccga gacacagtcg tgggcaccat gggcctgaag     480
   gccacgggcc ggctctgcac cgtggctaag gcaagggggc tgcgagcctg caggggagag     540
   ctgagggaca ccatcctaga ctgggaggac tccctgcccg accgggacct ggcactcgcc     600
   gatgaggcca gcaggaacgc cgacctgtcc atcacgctgg gtacatcgct gcagatccgg     660
   cccagcggga acctgccgct ggctaccaag cgccggggag gccgcctggt catcgtcaac     720
   ctgcagccca ccaagcacga ccgccatgct gacctccgca tccatggcta cgttgacgag     780
   gtcatgaccc ggctcatgaa gcacctgggg ctggagatcc ccgcctggga cggcccccgt      840
   gtgctggaga gggcgctgcc acccctgccc cgcccgccca cccccaagct ggagcccaag     900
   gaggaatctc ccacccggat caacggctct atccccgccg gccccaagca ggagccctgc     960
   gcccagcaca acggctcaga gcccgccagc cccaaacggg agcggcccac cagccctgcc    1020
   ccccacagac cccccaaaag ggtgaaggcc aaggcggtcc ccagctga                 1068

## Description

### Technical Field

The present invention is defined by the claims. The present disclosure generally relates to an agent using at least one selected from the group consisting of an imidazole dipeptide and a metabolite thereof as an active ingredient. The agent of the present disclosure can be used for improvement of cognitive function, improvement of mental function, anti-aging, and health maintenance. The present disclosure is useful in the fields of common food, health food, drug, beauty, health, and medical science.

### Background Art

Carnosine is a dipeptide consisting of β-alanine and histidine, and anserine is a dipeptide consisting of β-alanine and methylated histidine. It is known that both are contained in chicken etc. Carnosine and anserine have been investigated for their actions for promotion of skin metabolism (Patent document 1), autonomic nerve control (Patent document 2), and stress relieving (Patent document 3), as well as improvement of learning function and anti-anxiety (Patent document 4).

In recent years, things for living long with maintaining health, such as prevention of diseases accompanying aging and strengthening of immune activity, have come to attract growing interest. In many cases, aging is accompanied by decrease of cognitive function to a certain extent. Decrease of cognitive function is also caused by onset or advance of the Alzheimer disease.

### Prior art references

### Patent documents

Patent document 1: Japanese Patent Unexamined Publication (Kokai) No. 2000-201649
Patent document 2: WO2002/076455
Patent document 3: Japanese Patent Unexamined Publication (Kokai) No. 2007-70316
Patent document 4: Japanese Patent Unexamined Publication (Kokai) No. 2000-116987

### Summary of the Invention

### Object to be Achieved by the Invention

It is desirable to delay advance of aging in the daily life with appropriate meals, exercises, psychotherapies, etc, and manage diseases accompanying aging at an early stage.

Foods are energy sources and sources of essential nutrients (nutritiousness), and in addition, they also give pleasure of eating (preference) and contribute to healthy life (functionality). Foods and products containing food-derived functional ingredients are expected to be useful for health or beauty, and they are generally and widely accepted as health foods etc. It is considered that use of already habitually eaten natural products is preferred also from the viewpoints of safety and no anxiety.

### Means for Achieving the Object

The inventors of the present invention have worked on researches and developments of foods and materials useful for health by utilizing natural products. In this process, they recently found that carnosine and anserine derived from chicken had a mental function-improving action etc., and accomplished the present invention. The present invention provides the followings.
[1] An agent for use in preventing onset of dementia due to aging, which contains at least one imidazole dipeptide.
[2] An agent for use in suppressing a cerebral atrophy, which contains at least one imidazole dipeptide.
[3] The agent for use according to [1] or [2], wherein the agent is for ingestion of 200 mg or more as a daily dose of the at least one imidazole dipeptide.
[4] The agent for use according to any one of claims [1] to [3], wherein at least one kind of the imidazole dipeptide is derived from chicken.
[5] The agent for use according to any one of claims [1] to [4], which further contains creatine and a nucleic acid.
[6] The agent for use according to any one of claims [1] to [5], which is for an elderly person or a person with a slight mood disorder.

### Brief Description of the Drawings

[Fig. 1] Results of a mental function test (BDI test)
[Fig. 2] Results of a mental function test (ADAS-cog test for evaluating aging of brain): ratios of improvement, no change, and degradation are indicated.
[Fig. 3-1] Parts of brain (gray matter) in which atrophy was improved by ingestion of an imidazole dipeptide: parts that showed p < 0.005 are indicated as colored parts.
[Fig. 3-2] Parts of brain (white matter) in which atrophy was improved by ingestion of an imidazole dipeptide
[Fig. 4] Improvement of neural circuit function provided by ingestion of an imidazole dipeptide: although the neural circuit function of the hippocampus and posterior cingulate gyrus decreases in connection with aging (left), this decrease of function was improved in the test diet ingestion group after the ingestion (right).
[Fig. 5] Genes of which expression was changed by ingestion of an imidazole dipeptide: the gene expression levels of 6 genes belonging to SLC (transporter) were significantly changed by the ingestion of the test diet compared with that observed with ingestion of placebo food. In addition, it was also found that the expression levels of the other genes shown in the graph including chemokines genes also changed.
[Fig. 6] Blood cytokines and chemokines of which expressions were changed by ingestion of an imidazole dipeptide: the results are shown with average ± SE, the results of the test diet ingestion group are indicated with solid lines, the results of the placebo diet ingestion group are indicated with dashed lines, and the results of paired t-test are indicated with * (p < 0.05), and ** (p < 0.01).
[Fig. 7] Decrease of blood sugar level provided by ingestion of an imidazole dipeptide
[Fig. 8-1] Decrease of blood cytokines in dementia model mouse: in a carnosine-containing diet group, cytokines decreased, and it is suggested that inflammation was suppressed. The symbol wt stands for wild-type, tg for transgenic, * for p < 0.05, ** for p < 0.01 (Dunnett's test vs tgHFD), # for p < 0.05, and ## for p < 0.01 (Student t-test).
[Fig. 8-2] Suppression of intracerebral inflammatory reaction in dementia model mouse (mouse MRI images): suppression of inflammation provided by ingestion of an imidazole dipeptide was observed.
[Fig. 8-3] Suppression of expression of GABA transporter genes in glia cells provided by ingestion of highly functional dipeptide: Slc6A13 is GABA Transporter 2 (GAT-2) expressed in astrocytes, and Slc6A12 is Betaine/GABA Transporter 1 expressed in astrocytes (BGT-1).
[Fig. 8-4] Blood insulin levels in mice: between the values of the Alzheimer disease (AD) high fat diet group and the AD high fat diet + carnosine group, significant difference (student's t-test, p < 0.05) were observed.
[Fig. 9] ASL analysis
[Fig. 10] Parts for which differences were observed between the both groups in the ASL analysis (posterior cingulate gyrus)
[Fig. 11] Scores for logical memory II (results of subanalysis for those 60 years old or older): there was significant difference between both groups (p < 0.01). Difficulty of the test performed after ingestion was far higher than that of the test performed before ingestion.
[Fig. 12] Scores for logical memory II (results of subanalysis for those 60 years old or older): function was not degraded for all the ages in the test diet group, whereas there was a tendency that severer hypofunction was observed for higher age in the placebo diet group.

### Modes for Carrying out the Disclosure

### [Active ingredient]

The present disclosure relates to an agent that uses at least one selected from the group consisting of an imidazole dipeptide and a metabolite thereof as an active ingredient. The term imidazole dipeptide used in this specification refers to a dipeptide consisting of an amino acid having an imidazole ring, and another amino acid, which bind together, unless especially mentioned.

The imidazole dipeptide as used herein can be represented by following formula I or II.

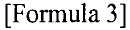

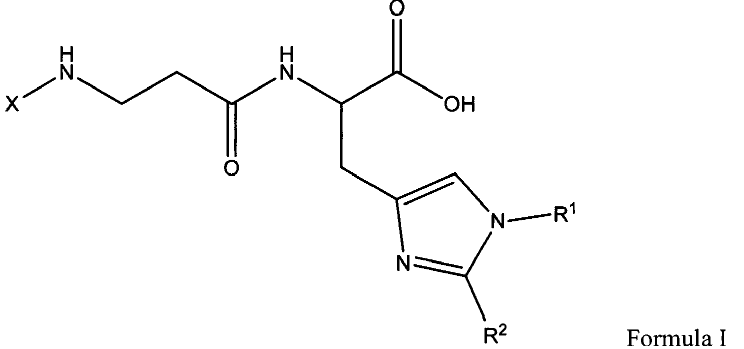

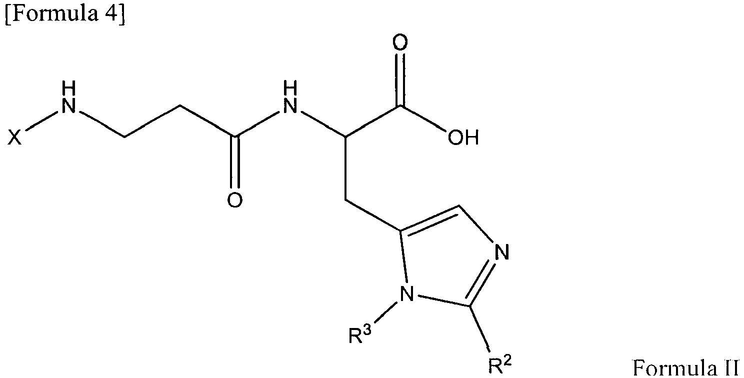

In the formulas I and II, R¹, R², and R³ are independently H or a C₁₋₆ alkyl, and X is H or -COR⁴, wherein R⁴ is H, a C₁₋₆ alkyl, benzyl which may be substituted, or H₂C=CH-.

In the formula I, it is preferred that one of R¹ and R² is a C₁₋₆ alkyl, and the other is H. In the formula II, it is preferred that one of R² and R³ is a C₁₋₆ alkyl, and the other is H. One of the preferred examples of C₁₋₆ alkyl is methyl.

Specific examples of -COR₄ as X include formyl, acetyl, propionyl, benzoyl, and acryloyl.

As for methods for producing the compounds represented by the formula I or II, Japanese Patent Unexamined Publication (Kohyo) No. 2003-520221, Japanese Patent Unexamined Publication (Kokai) No. 2006-232686, Japanese Patent Unexamined Publication (Kohyo) Nos. 2006-504701, 2008-517911, 2009-512459, Japanese Patent Unexamined Publication (Kokai) Nos. 2010-31004, 2011-37891, 2011-37892, 2013-165728, 2014-12735, can be referred to.

Carnosine, anserine, balenine, and homocarnosine are included in the scope of the imidazole dipeptide. Carnosine is a dipeptide consisting of β-alanine and histidine. Depending on the conformation of the constituent histidine, carnosine exists as L-isomer or D-isomer. In the present disclosure and explanations thereof, when only the term "carnosine" is used, it refers to L-carnosine, D-carnosine, or a mixture of them, unless especially indicated. It is known that L-carnosine exists in muscles and nervous tissues of mammals such as human at comparatively high concentrations.

The structure of L-carnosine (IUPAC nomenclature: (2S)-2-[(3-amino-1-oxopropyl)amino]-3-(3H-imidazol-4-yl)propanoic acid) is shown below.

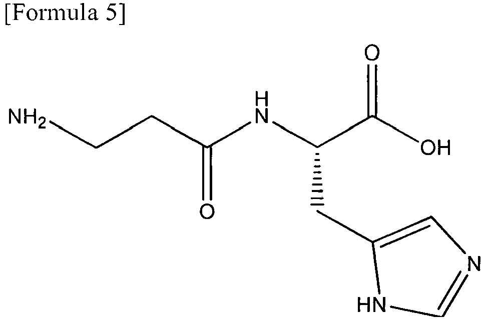

L-Anserine, which consists of β-alanine and methylated histidine, is abundantly observed in some animals. In the present disclosure and explanations thereof, when only the term "anserine" is used, it refers to L-anserine, D-anserine, or a mixture of them, unless especially indicated. The structure of L-anserine (IUPAC nomenclature: (2S)-2-[(3-amino-1-oxopropyl)amino]-3-(3-methyl-4-imidazolyl)propanoic acid) is shown below.

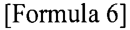

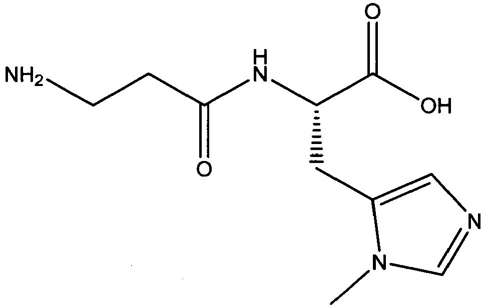

Both of carnosine and anserine are water-soluble (1 g/3.1 ml at 25°C for carnosine).

The term metabolite of imidazole dipeptide used in this specification refers to one kind of metabolite selected from the group consisting of metabolites of carnosine, anserine, balenine, and homocarnosine, unless especially indicated. β-Alanine, histidine, methylated histidine, and γ-aminobutyric acid (GABA) are included in the scope of the metabolite of imidazole dipeptide.

The expression "at least one selected from the group consisting of an imidazole dipeptide and a metabolite thereof" used in the present disclosure is used to mean one or two or more kinds of imidazole dipeptides, metabolites thereof or both, unless especially indicated. For example, the expression "to contain at least one selected from the group consisting of an imidazole dipeptide and a metabolite thereof as an active ingredient" means, for example, to contain carnosine as an active ingredient, but not contain any other imidazole dipeptides, to contain carnosine and anserine as active ingredients, or the like. When quantity or concentration is referred to for "at least one selected from the group consisting of an imidazole dipeptide and a metabolite thereof', and there are two or more kinds of imidazole dipeptides, metabolites thereof or both, the quantity or concentration refers to the total quantity or concentration of all the imidazole dipeptides, metabolites thereof or both. Although an explanation may be made in this specification for an embodiment using an imidazole peptide, or carnosine or anserine among the imidazole dipeptides and metabolites thereof, such an explanation also applies to implementations using another imidazole dipeptide or a metabolite thereof.

In the present disclosure, the imidazole dipeptide and a metabolite thereof used as the active ingredient may be a synthesized one or one produced by fermentation, or one obtained from a natural product. They may be an isolated one, or purified one. More specifically, the imidazole dipeptide and a metabolite thereof may be one derived from any of various animals such as bovine, equine, porcine, fowl, whale, and fish (such as bonito, tuna, and eel). One of the preferred examples is one derived from chicken. The imidazole dipeptide or a metabolite thereof may be contained in the agent as an extract, concentrate, roughly purified product, or the like of a natural product.

### [Use and function]

The agent of the present disclosure can be used for improvement of a neuropsychologic function. The improvement of a neuropsychologic function includes antidepressing (improvement of mental function) and improvement of cognitive function. The improvement of a neuropsychologic function also includes suppression of cerebral atrophy, suppression of cerebral hypofunction (enhancement of functional connectivity to the hippocampus), and improvement of damage of neurons caused by inflammation, which relate to a neuropsychologic function. The neuropsychologic function may be one relating to the Alzheimer disease or aging. The improvement of a neuropsychologic function also includes a treatment of aging of cerebral function and/or dementia.

The antidepressing (mental function-improving) effect provided by the agent of the present disclosure can be estimated by using the BDI questionnaire (http://www.chibatc.co.jp/catalogue/04/1/67.html). Since a high score of the BDI questionnaire represents a depressing tendency, degree of the improvement of a neuropsychologic function can be estimated by, for example, performing the inquiry before and after the ingestion of the agent of the present disclosure, and comparing the scores obtained before and after the ingestion. An effect of the agent of the present disclosure for improving a mental function can be expected especially for a subject having a slight mood disorder.

The effect of the agent of the present disclosure for improving cognitive function can be estimated by the ADAS-cog (Alzheimer's Disease Assessment Scale-cognitive subscale) method. The effect of the agent of the present disclosure for improving cognitive function includes those for improving the function degraded to such a degree that memory worsens with aging, and such a degree that cognitive ability degrades pathologically (dementia). The improving effect of the agent of the present disclosure can be expected especially for degradation of cognitive function relating to the Alzheimer disease or aging.

The agent of the present disclosure can be used for suppression of cerebral atrophy, suppression of cerebral hypofunction (enhancement of functional connectivity to the hippocampus), or improvement of damage of neurons caused by inflammation. The effects on these can be evaluated by the methods well known to those skilled in the art, such as diagnostic imaging. According to the examination of the inventors of the present disclosure, in a group of subjects including elderly persons, parts where advance of atrophy was suppressed were observed in both the cerebral gray matter and white matter. Such an effect was not observed in a group for which a placebo not containing anserine and carnosine was administered.

The agent of the present disclosure can be used for treating aging of cerebral function and onset of dementia, more specifically, for preventing, delaying, or inhibiting aging of cerebral function and onset of dementia, or preventing aggravation of these. Such an effect can be confirmed by evaluating blood flow change in the posterior cingulated gyrus of the subject, or evaluating logical memory (delayed word recall task) in the case of a subject 60 years old or older. According to the investigation of the inventors of the present disclosure, it was found that, in the case of subjects 60 years old or older, blood flow was maintained in the posterior cingulated gyrus in the imidazole dipeptide-containing test diet ingestion group in a degree significantly different from that observed for the placebo diet group. It was also found that, in the case of subjects 60 years old or older, the scores for the logical memory were maintained in the group of the subjects who ingested test diet for 3 months in a degree significantly different from that observed for the placebo diet group.

The agent of the present disclosure can be used for changing expression of a transporter. Transporters are membrane proteins existing on a cell membrane together with a channel or receptor. However, unlike channel, transporters recognize not only an endogenous substance, but also many exogenous substances including drugs and environmental chemical substances as a transportation substrate. Transporters are classified into two groups, ABC (ATP binding cassette) family, of which members carry out the transportation by using hydrolysis energy of ATP, and SLC (solute carrier) family, of which members carry out the transportation without using the energy of ATP, and 48 kinds of ABC transporter genes and 319 kinds of SLC transporter genes have been identified for human. Diseases caused by abnormalities of transporters increase with aging, and it is supposed that transporters relate to about 10% of genes relating to aging-associated diseases observed after 50 years old.

The agent of the present disclosure can be used especially for changing expression of at least one, preferably three or more, more preferably 5 or more, transporters selected from the group consisting of SLC23A2, SLC43A2, SLC29A3, SLC35C1, SLC25A33, SLC25A23, SLC6A12, and SLC6A13, further preferably all of them. Changing expression includes increasing expression and decreasing expression.

The agent of the present disclosure can be used for changing expression of a chemokine. Chemokines are basic proteins that exhibit the actions thereof via a G-protein-coupled receptor, and constitute a group of cytokines. They induce migration of leucocytes, and so forth, and participate in formation of inflammation. Many chemokines have been discovered so far. According to structural difference, they are classified into CC chemokines, CXC chemokines, C chemokines, and CX3C chemokines. Not less than 50 kinds of chemokines have been identified so far.

The agent of the present disclosure can be used especially for changing expression of one selected from the group consisting of CXCL12 and CCL17, preferably both chemokines. Changing expression includes increasing expression and decreasing expression.

The agent of the present disclosure can be used for changing expression of an aging-related gene. Aging-related genes have been identified as genes involved in individual aging or cellular aging, and many aging-related genes have been identified so far.

The agent of the present disclosure can be used especially for changing expression of one selected from the group consisting of TSPO and P2RY1, preferably both aging-related genes. Changing expression includes increasing expression and decreasing expression.

The agent of the present disclosure can be used for changing expression of a nervous system gene. Nervous system genes are genes involved in neurogenesis, nerve differentiation, and so forth, and many nervous system genes have been identified so far.

The agent of the present disclosure can be used especially for changing expression of the nervous system gene, CAMK1. Changing expression includes increasing expression and decreasing expression.

The agent of the present disclosure can be used for changing expression of a mitochondrial gene. Mitochondrial genes are genes involved in mitochondrial biosynthesis, fusion, TCA cycle, and respiration, and many mitochondrial genes have been identified so far.

The agent of the present disclosure can be used especially for changing expression of at least one, preferably 3 or more, more preferably 5 or more, mitochondrial genes selected from the group consisting of ACO2, ATP7A, POLG, IDH3G, UCP2, BCKDHA, and TAP2, further preferably all of them. Changing expression includes increasing expression and decreasing expression.

The agent of the present disclosure can be used for changing expression of an anti-aging gene. Anti-aging genes are genes that realize suppression of cellular aging and anti-aging, and many such genes have been identified.

The agent of the present disclosure can be used especially for changing expression of one selected from the group consisting of SMARCD1 and SIRT6, preferably both of them. Changing expression includes increasing expression and decreasing expression.

The agent of the present disclosure can also be used for controlling at least one kind of cytokine selected from the group consisting of IP-10 (CXCL10), IL-2, IL-5, IL-7, IL-8 (CXCL8), IL-13, G-CSF, and MCP-1 (CCL2). The controlling includes increasing the level and decreasing the level.

The agent of the present disclosure can also be used as an anti-inflammatory agent, or for suppressing increase of blood sugar level or decreasing the level.

The "improvement" or "treatment" referred to in the present disclosure for a disease or condition includes reducing risk of onset, delaying, preventing or treatment of onset, and arresting or delaying advance. Practices for the improvement or treatment include medical practices aiming at treatment of diseases performed by medical practitioners, and non-medical practices performed by those other than medical practitioners, such as dietitian (registered dietitian), hygienist, maternity nurse, nurse, medical technologist, cosmetic adviser, aesthetician, food manufacturer, and food vender. The treatment includes recommendation of administration or ingestion of specific foods, guidance for food ingestion method, health guidance, nutrition guidance (including guidance for nutrition required for medical treatment of sick persons, and guidance for nutrition for maintenance and promotion of health), food services, and guidance required for nutritional improvement relating to food dispensing. Objects of the treatment according to the present disclosure include humans (individuals), preferably humans who are desirably to be subjected to any one of the aforementioned treatments, or need to be subjected to any one of the aforementioned treatments.

The inventors of the present invention measured serum carnosine decomposition enzyme (henceforth referred to as CNDP1) activity of subjects for the purpose of verifying individual differences that affect the efficacy of imidazole dipeptide. As a result of the activity measurement, it was confirmed that there were significant individual differences among the subjects. CNDP1 exists in blood, and decomposes an imidazole dipeptide. Therefore, CNDP1 may affect the imidazole dipeptide concentration in blood after ingestion of the imidazole dipeptide, and thereby affect the efficacy of the imidazole dipeptide. Therefore, it is thought that information of the activity of CNDP1 in a subject is useful for judging beforehand whether the treatment by ingestion of an imidazole dipeptide is effective or not. Therefore, the present disclosure provides a method for determining a neuropsychologic function of a subject based on the carnosine decomposition enzyme (CNDP1) activity of the subject, and a method for predicting a neuropsychologic function-improving effect provided to a subject by making the subject ingest the agent or nutritional composition of the present disclosure based on the CNDP1 activity of the object. These methods may be such a method in which a standard value for judgment is defined beforehand, and the judgment or prediction is mechanically performed using the standard value.

### [Agent]

The term "agent" used in the present disclosure may refer to the active ingredient itself, or one containing the active ingredient and another ingredient, unless especially indicated. However, it does not include existing foods containing at least one selected from the group consisting of an imidazole dipeptide and a metabolite thereof, such as chicken itself.

The agent of the present disclosure may contain an ingredient other than the active ingredient, so long as it can exhibit the objective effect. The other ingredient may be any of various additives acceptable for foods, and various additives acceptable for drugs. Examples of such an ingredient include excipients, antioxidants (antioxidizing agents), perfumes, seasonings, sweeteners, coloring agents, thickening stabilizers, color developing agents, bleaching agents, antifungal agents, gum bases, bitter taste agents, enzymes, brighteners, acidulants, emulsifiers, enhancers, agents for manufacture, binders, tension agents (isotonic agents), buffering agents, dissolving aids, preservatives, stabilizers, coagulants, and so forth.

The other ingredient may be a functional ingredient other than the active ingredient. Examples of such a functional ingredient include amino acids (for example, branched chain amino acids, ornithine), unsaturated fatty acids (for example, EPA, DHA), vitamins, trace metals, glucosamine, and chondroitins.

When the agent of the present disclosure consists of the active ingredient and another ingredient other the active ingredient, content of the active ingredient can be appropriately determined by those skilled in the art from the viewpoints of ease of manufacture, ease of use, etc., and it may be, for example, 0.1 to 99.9%, 1 to 95%, 10 to 90%, or 51 to 90%. Content of carnosine may be 21% or higher, and content of anserine may be 31% or higher.

As described above, form of the agent of the present disclosure may be any of various forms other than those of existing foods. For example, it may be a pharmaceutical composition such as an oral drug, or nutritional composition. The agent of the present disclosure can be used by adding it to a pharmaceutical composition such as an oral drug, or nutritional composition. The "nutritional composition" referred to in the present disclosure includes not only a solid one, but also one in the form of liquid, such as drink, unless especially indicated. The "nutritional composition" referred to in the present disclosure also includes health food, supplement, and food with health claims (including food with nutrient function claims and food for specified health uses), as well as dietetic food (one that achieves the purpose of treatment, which is cooked according to a menu prepared by a dietitian or the like according to a dietary slip prepared by a medical practitioner), food for alimentary therapy, homogenized food, low salt diet, care food, decreased calorie diet, diet food, and materials for these, unless especially indicated.

Examples of the form of the agent of the present disclosure, the pharmaceutical composition, and the nutritional composition include powder, subtilized granule, granule, tablet, capsule, liquid preparation (including elixir, lemonade, syrup, emulsion, suspension, solution, and drinkable preparation), gelatinous formulation, dietetic food, drink, confectionery, meat product, marine product-processed product, vegetable-processed product, daily dish, seasoning composition, and food additive.

Ingestion amount of the active ingredient of the present disclosure can be appropriately determined by those skilled in the art according to age, weight, sex, disease or condition to which the agent is applied, and so forth of the subject who ingests it. Ingestion amount of the active ingredient may be, for example, 200 mg/day or more, preferably 400 mg/day or more, more preferably 500 mg/day or more, further preferably 750 mg/day or more. It may also be 1,000 mg/day or more, 2,000 mg/day or more, 5,000 mg/day or more, or 7,500 mg/day or more. In any case, it can be 10,000 mg/day or less. Irrespective of how the minimum amount is defined, it may be 50,000 mg/day or less, preferably 30,000 mg/day or less, more preferably 20,000 mg/day or less, more preferably 10,000 mg/day or less. The active ingredient of the aforementioned daily ingestion amount may be ingested at one time, or at a plurality of times as divided portions.

Although the amount of the active ingredient contained in the agent, pharmaceutical composition, or nutritional composition of the present disclosure can be appropriately determined by those skilled in the art, it may be, for example, 1,000 mg/100 g or more, preferably 1,500 mg/100 g or more, more preferably 2,000 mg/100 g or more, still more preferably 2,500 mg/100 g or more, further preferably 3,000 mg/100 g or more, still further preferably 3,500 mg/100 g or more. Irrespective of the minimum amount, it may be 50,000 mg/100 g or less, preferably 40,000 mg/100 g or less, more preferably 30,000 mg/100 g or less, further preferably 20,000 mg/100 g or less.

The agent, pharmaceutical composition, or nutritional composition of the present disclosure may also contain an ingredient other than the active ingredient. The ingredient other than the active ingredient is, for example, creatine or a nucleic acid. Content of creatine can be, for example, 10 mg or more, preferably 20 mg more, more preferably 30 mg or more, still more preferably 60 mg or more, further preferably 100 mg or more, still further preferably 200 mg or more, in the daily dose. Irrespective of the minimum amount, it can be 2,000 mg or less, preferably 1,000 mg or less, more preferably 750 mg or less, further preferably 500 mg or less. Content of a nucleic acid can be, for example, 0.15 mg or more, preferably 0.30 mg or more, more preferably 0.50 mg or more, still more preferably 1.0 mg or more, further preferably 2.0 mg or more, still further preferably 3.0 mg or more, in the daily dose. Irrespective of the minimum amount, it can be 50 mg or less, preferably 40 mg or less, more preferably 20 mg or less, further preferably 10 mg or less.

When the agent, pharmaceutical composition, or nutritional composition of the present disclosure is used as a dietetic food (one that achieves the purpose of treatment, which is cooked according to a menu prepared by a dietitian or the like according to a dietary slip prepared by a medical practitioner), food for alimentary therapy, homogenized food, low salt diet, care food, decreased calorie diet, diet food, or sports food (including food aiming at enhancement of ability in aerobic exercise, food aiming at enhancement of endurance in aerobic exercise, food for accumulating nutrition in the body by the day of game, food for supplementing nutrition during game, and food aiming at recovery from exhaustion after finishing game), content of the active ingredient can be determined in consideration of ingestion amount as one meal.

The agent, pharmaceutical composition, or nutritional composition of the present disclosure can be ingested by a subject repeatedly or over a long period of time. Especially when enhancement of exercise capacity is intended, it will be preferable to make the subject ingest it before exercise, or ingest daily.

For the agent, pharmaceutical composition, or nutritional composition of the present disclosure, it can be indicated that it can be used for improvement of a neuropsychologic function, suppression of cerebral atrophy, suppression of cerebral hypofunction, and improvement of damage of neurons caused by inflammation, and it can also be indicated that ingestion thereof is recommended to, for example, elderly people 65 years old or older, or persons having a slight mood disorder. The indication may be a direct indication, or an indirect indication. Examples of the direct indication include a statement on articles such as the product itself, package, container, label, and tag, and examples of the indirect indication includes advertising and public relations on such places or by such means as website, shop front, exhibition, signboard, bulletin board, newspaper, magazine, television, radio, postal matter, and E-mail.

### [Production method]

The agent, pharmaceutical composition, or nutritional composition of the present disclosure can be produced by using various known techniques. The step of adjusting concentration of the active ingredient to a predetermined concentration may be performed in various stages of the manufacturing process. Those skilled in the art can appropriately design the steps for producing the agent of the present disclosure in consideration of solubility, stability, volatility, etc. of the active ingredient. According to the investigations of the inventors of the present disclosure, it was confirmed that anserine and carnosine are sufficiently stable at an ordinary temperature, and they are also sufficiently stable under the cooking conditions of 180°C or lower. It was also confirmed that they can be stably stored over at least 2 years and 9 months in a state of solution.

When the active ingredient of the present disclosure is constituted as a chicken extract, an example of the method for producing such a chicken extract specifically comprises mincing chicken, adding warm water to the chicken, adjusting pH of the mixture if needed, and allowing extraction over several minutes to several days with warming as required. As for the extraction conditions, the treatment is performed, for example, at 50 to 100°C for 1 to 10 hours. The obtained extract can be purified or fractionated by diatomaceous earth filtration, ultrafiltration, or the like, as required. The product can be subjected to a demineralization treatment and protease treatment as required. Although the part from which the chicken as the raw material is obtained is not particularly limited, it preferably contains breast meat, since it abundantly contains carnosine and/or anserine. The obtained extract can be dried into a dry product by hot air drying, spray drying, lyophilization, or the like. It can also be granulated into granules.

### [Expression analysis]

The present disclosure also provides a method for detecting improvement or degradation of a neuropsychologic function, which is based on analysis of expression of at least one kind of gene selected from the group consisting of transporter genes SLC23A2, SLC43A2, SLC29A3, SLC35C1, SLC25A33, SLC25A23, SLC6A12, and SLC6A13; chemokine genes CXCL12 and CCL17; aging-related genes TSPO and P2RY1; nervous system gene CAMK1; mitochondrial genes ACO2, ATP7A, POLG, IDH3G, UCP2, BCKDHA, and TAP2; as well as anti-aging genes SMARCD1 and SIRT6, and a kit for detecting improvement or degradation of a neuropsychologic function, which comprises a nucleic acid comprising at least one kind of nucleotide sequence selected from an entire or partial sequence of any one of the nucleotide sequences of SEQ ID NOS: 1 to 20, and an entire or partial sequence of a nucleotide sequence complementary to any one of the nucleotide sequences of SEQ ID NOS: 1 to 20.

The expression analysis is preferably performed by analyzing expression of at least one kind of transporter gene selected from the group consisting of SLC23A2, SLC43A2, SLC29A3, SLC35C1, SLC25A33, SLC25A23, SLC6A12, and SLC6A13, at least one kind of chemokine gene selected from the group consisting of CXCL12 and CCL17, at least one kind of aging-related gene selected from the group consisting of TSPO and P2RY1, a nervous system gene selected from CAMK1, at least one kind of mitochondrial gene selected from the group consisting of ACO2, ATP7A, POLG, IDH3G, UCP2, BCKDHA, and TAP2, and at least one kind of anti-aging gene selected from the group consisting of SMARCD1 and SIRT6. More preferably, the expression analysis is performed by analyzing expression of all of these genes.

In the development of ingredients of new drugs, functional food compositions, etc., actions of candidate drugs or ingredients are monitored at the cellular level to evaluate efficacy and safety thereof, and a technique of quantifying genes expressed in cells before and after administration of a drug or ingredient for the entire genome region, and quantitatively grasping action of the drug or ingredient as change of expression amounts of the genes attracts attentions. By analyzing expressions of a combination of genes defined in the present disclosure using such a method as mentioned above, effects of a candidate drug on a neuropsychologic function can be analyzed.

In the aspects of the present disclosure concerning expression analysis, the nucleic acid comprising at least one kind of nucleotide sequence selected from an entire or partial sequence of any one of the nucleotide sequences of SEQ ID NOS: 1 to 20, and an entire or partial sequence of a nucleotide sequence complementary to any one of the nucleotide sequences of SEQ ID NOS: 1 to 20 may be a probe that can specifically hybridize with a transcript in a sample as an object of detection, or a pair of primers that can function as primers for amplifying all or a part of such a transcript as mentioned above. The nucleic acid may be DNA or RNA.

In the aspects of the present disclosure concerning expression analysis, length of the nucleic acid comprising at least one kind of nucleotide sequence selected from an entire or partial sequence of any one of the nucleotide sequences of SEQ ID NOS: 1 to 20, and an entire or partial sequence of a nucleotide sequence complementary to any one of the nucleotide sequences of SEQ ID NOS: 1 to 20 used as the probe is, for example, 15 nucleotides length or longer, preferably 20 nucleotides length or longer, more preferably 25 nucleotides length or longer. In order to enable detection and quantification of a target nucleic acid, the probe nucleic acid may be labeled with, for example, a radioisotope, enzyme, fluorescent substance, or luminescent substance. The nucleic acid used as the probe may be immobilized on a solid phase.

Length of the nucleic acids used as primers is, for example, 15 to about 100 nucleotides length, preferably 15 to 50 nucleotides length, and they preferably consist of a pair of nucleotide sequences designed so that they can amplify a DNA fragment of 100 bp to several kbp.

A nucleic acid to be used can be prepared by chemical synthesis using a commercial automatic DNA/RNA synthesizer, or the like. A chip (array) on which a nucleic acid is immobilized can also be prepared by synthesizing the nucleic acid directly on a solid phase of silicon, glass, or the like. Preferred examples of implementation in which the nucleic acid probe is immobilized on a substrate include a DNA micro array.

For quantitatively analyzing expression of predetermined genes using a small amount of sample, competitive RT-PCR or real-time RT-PCR can be used. The sample as the object of the analysis may be blood collected from human.

### [Use as leading compound]

The present disclosure provides a method for searching for an active ingredient or the like for improvement of a neuropsychologic function, especially a treatment of aging of a cerebral function, and/or dementia, etc. (screening method) by using the aforementioned imidazole dipeptide or a metabolite thereof as a leading compound. The term leading compound generally refers to a compound of which profiles of pharmacological activities have been clarified, and for which improvement of activity or reduction of toxicity is expected as a result of chemical modification thereof. The imidazole dipeptide and a metabolite thereof have pharmacological activities for improvement of a neuropsychologic function, especially a treatment of aging of a cerebral function and/or dementia as described above, and it is expected that improvement of the activity or reduction of toxicity is provided by chemical modification thereof.

The chemical modification means, for example, optimization of a leading compound through chemical modification of the leading compound. The chemical modification can be, for example, substitution or deletion of a part of amino acids, or addition or insertion of at least one amino acid. There are also contemplated addition, substitution or removal of a functional group on an amino acid, and substitution of D-amino acid or artificial amino acid for an amino acid.

By performing optimization using the imidazole dipeptide or a metabolite thereof as a leading compound according to the present disclosure, an active ingredient showing further superior physicochemical properties, pharmacokinetics, toxicity, etc. can be searched for.

Hereafter, the present invention will be explained with reference to examples. However, the scope of the present invention is not limited by the following examples.

### Examples

### [Evaluation 1 with healthy volunteers]

Test diets containing chicken-derived imidazole dipeptides (1000 mg as daily dose of carnosine and anserine) were given to test subjects (28 healthy male or female volunteers 40 years old or older, divided into two groups of test diet group and placebo diet group) over 3 months, and change of the cerebral function etc. were evaluated before, during, and after the test period. Compositions of the test diet and placebo diet are shown in the following tables (as daily amounts).

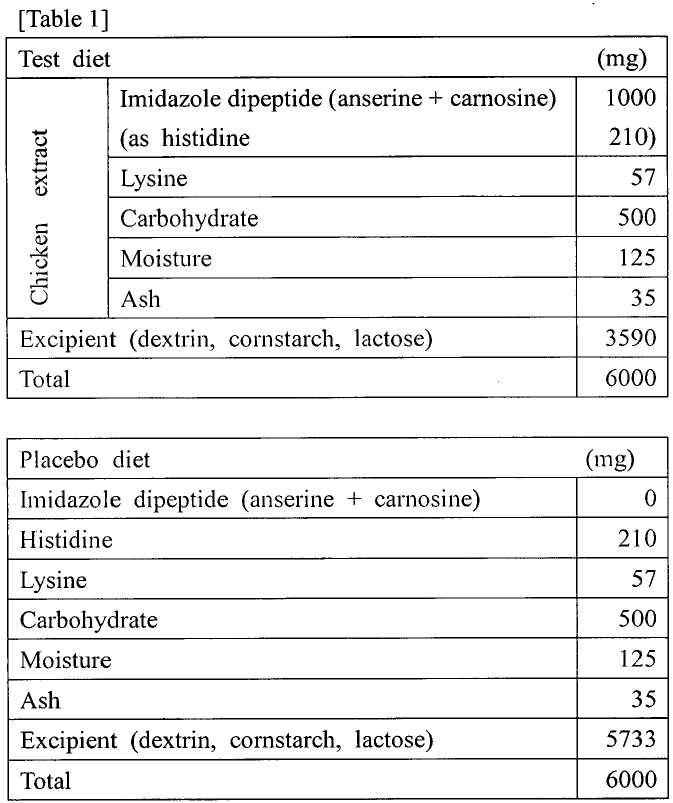

### 1. Antidepressing effect (evaluation using BDI questionnaire)

Before and after the ingestion period, depressing tendency was evaluated with the BDI questionnaire (http://www.chibatc.co.jp/catalogue/04/1/67.html). A higher score of the BDI questionnaire indicates higher depressing tendency.

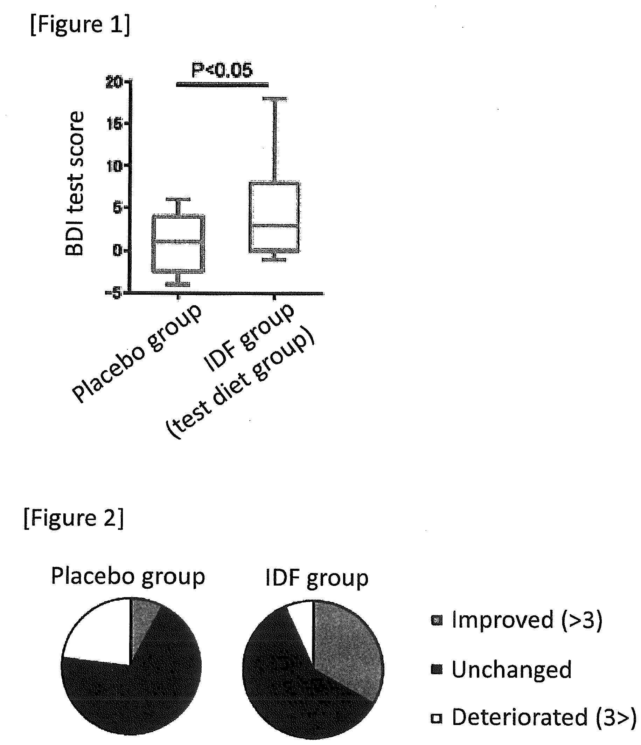

The results are shown in Fig. 1. Degrees of the improvement were compared on the basis of change of BDI score, i.e., score obtained before ingestion (test 1) - score obtained after ingestion (test 2). As a result, improvement tendency was seen for the test diet group, whereas improvement was hardly seen for the placebo group. Further, the changes before and after ingestion were ranked (a larger number of ranking indicates higher improvement). As a result, there was observed a tendency that the test diet group showed larger change. Even healthy subjects may have a slight depressing tendency, and it is considered that the imidazole dipeptide improved such a tendency by making functions of the GABA nervous system complete.

### 2. Cognitive function-improving effect (Evaluation using ADAS-cog)

Cognitive functions were evaluated before and after the ingestion period by using ADAS-cog (Alzheimer's Disease Assessment Scale-cognitive subscale).

The results are shown in Fig. 2. Ratios of the subjects whose scores were improved or degraded by 3 points or more are shown. The ratio of subjects whose scores were improved of the test diet group was larger than that of the placebo group.

### 3. Effect on cerebral atrophy etc.

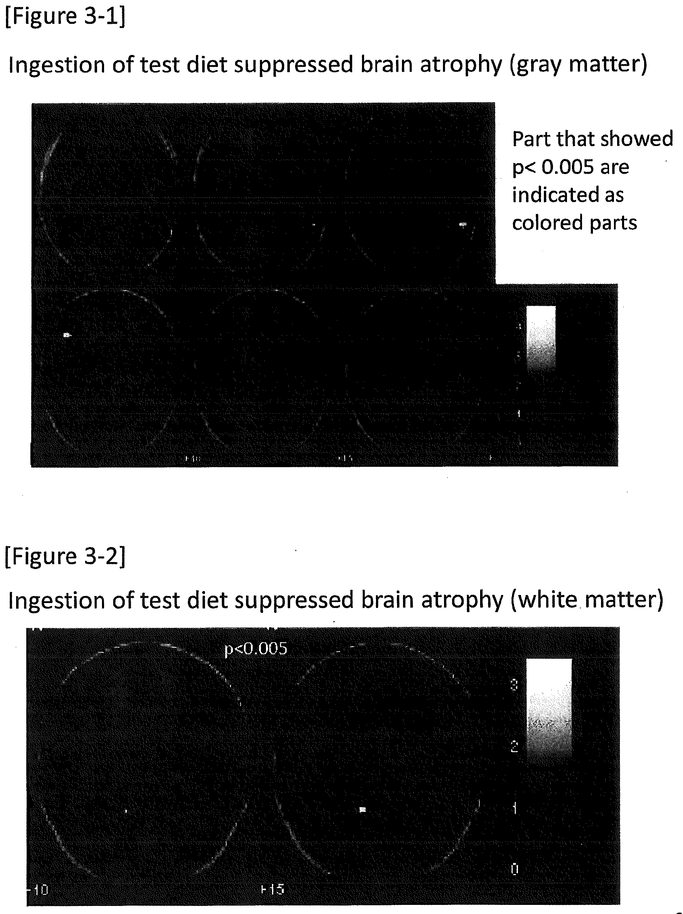

Brain structure analysis based on three-dimensional T1-weighted image and functional connectivity analysis based on resting-state functional MRI were performed. As a result of longitudinal analysis for base line and structural change after three months performed for 15 subjects of the test diet group and 13 subjects of the placebo diet group, it was found that advance of atrophy was more suppressed in the test diet group compared with the placebo diet group in the right inferior frontal gyrus and left inferior temporal gyrus for the gray matter (Fig. 3-1), and in the right posterior cingulate gyrus for the white matter (Fig. 3-2).

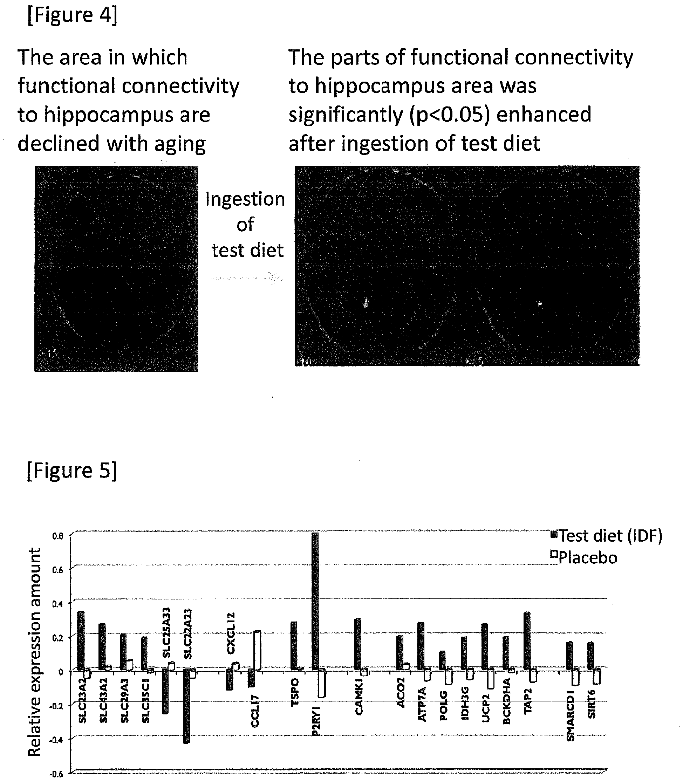

In the functional connectivity analysis based on resting-state functional MRI, it was found for the baseline that the functional connectivity to the hippocampus was reduced in the posterior cingulate gyrus with aging (Fig. 4). It is known that the posterior cingulate gyrus participates in reproduction of memory, and the function thereof reduces first in the Alzheimer disease. In the test diet group, the functional connectivity to the hippocampus of this part was enhanced three months afterward compared with the placebo diet group. This part coincided with the part of the white matter where the atrophy-suppressing effect was seen in the test diet group (Fig. 3-2).

### 4. Gene expression analysis

Blood samples of 13 subjects of the test diet group (samples could not be prepared for 2 subjects) and all the 13 subjects of the placebo group (those at the time of the first test and midterm test) were collected by using PAXgene RNA blood collection tubes (Nippon Becton Dickinson Company, Ltd., Tokyo), high-quality RNA was prepared by using PAXgene Blood RNA kit (Qiagen), and change of gene expression was analyzed by using a microarray.

### Methods

Whole Human Genome Oligo DNA Microarray (4x44K) v2 (Agilent, CA, USA) was used as microarray.

### (1) Labeling

First, total RNA was extracted from each blood sample of test subject by using PAXgene Blood RNA Kit (Qiagen), and 200 ng of each total RNA was labeled by using Agilent Low-Input QuickAmp Labeling Kit, One-color. First, 200 ng of the total RNA in a volume of 2.5 µL was added to 2 µL of One-Color Spike Mix stock solution prepared beforehand. Then, 0.8 µL of T7 Promoter Primer was added, the mixture was incubated at 65°C for 10 minutes on a heat block, and then quenched on ice for 5 minutes. Then, 4.7 µL of cDNA Master Mix prepared beforehand was further added, and the mixture was incubated on a heat block at 40°C for 2 hours, and moved onto a heat block at 70°C for further incubation for 15 minutes. Then, the mixture was quenched for 5 minutes on ice, and 6 µL of Transcription Master Mix prepared beforehand was added to the mixture. The mixture was incubated for 2 hours on a heat block at 40°C with light shielding, then the total volume of the mixture was made to be 100 µL by addition of 84 µL of nuclease-free water, 350 µL of Buffer RLT was further added to the mixture, and 250 µL of ethanol was further added to the mixture. Then, the total volume of the mixture was applied to an RNeasy column, centrifuged at 4°C and 13000 rpm for 30 seconds, washed twice with 500 µL of a buffer RPE, and finally eluted with 30 µL of RNase-free water.

### (2) Hybridization

Then, hybridization was performed according to the protocol recommended by Agilent. First, RNA eluted above was fragmented by mixing it with Fragmentation mix, incubated for 30 minutes on a heat block at 60°C, and immediately cooled on ice for 1 minute. Then, cRNA from Fragmentation Mix was mixed with 2 x GE x Hybridization Buffer HI-RPM to prepare a hybridization mix. The hybridization mix was applied to the microarray slide, the slide was disposed in a hybridization chamber, and then disposed in a hybridization oven, and hybridization was allowed at 65°C and 10 rpm for 17 hours.

### (3) Washing and scanning of microarray slide

The microarray slide was washed by using Gene Expression Wash Buffer prepared beforehand. First, before the end of the hybridization, Gene Expression Wash Buffer 1 was filled in two glass containers for washing, and Gene Expression Wash Buffer 2 at 37°C was filled in another one glass container (total three glass containers for washing). After the end of the hybridization, the hybridization chamber was disassembled in the first glass container for washing, and the microarray slide was taken out, and washed in the second glass container for washing. The microarray slide was further washed in the third glass container for washing, then slowly pulled up from the water surface so that it was dried, and finally mounted on a scanner for exclusive use, and the microarray slide was scanned.

### (4) Data analysis

Data were converted into numerals with Feature Extraction software of Agilent. Normalization was performed according to the quantile method by using the statistical analysis software R. Z-Scores and ratios were calculated from the normalized signal values, and signal values showing variation of ±2 or larger were extracted. The obtained data were analyzed by using the annotation database DAVID (http://david.abcc.ncifcrf.gov/).

First, GenBank Accession Numbers of genes for which change was confirmed were inputted into the database, and then clustering was performed for every function for which genetic change occurred by performing functional annotation clustering. Pathway analysis of KEGG (Kyoto Encyclopedia of Genes and Genomes) was similarly conducted by using DAVID.

### Results

Genes that showed significant change due to the ingestion of the test diet compared with the placebo diet group are shown in Fig. 5, which were determined on the basis of significant difference level p < 0.05. From the results of this gene expression analysis, it was found that expressions of various kinds of transporter molecules existing on blood cells significantly changed. It was found that, in particular, expression amount of the vitamin C transporter, which exists on the membrane surface of lymphocyte, was significantly increased by the ingestion of the imidazole dipeptide (SLC23A2 in Fig. 5). Expressions of a plurality of genes relating to energy metabolism of mitochondria (ACO2 (aconidase) and IDH3G (isocitrate dehydrogenase), which are enzymes of TCA cycle) were increased. The imidazole dipeptide may exhibit the health promotion action by using such a mechanism.

As for the chemokines, decreases of expressions of the CXC chemokines and CC chemokines were observed. It is suggested that the test diet tends to suppress inflammation.

Enhancement of expressions of aging-related genes was also observed. Suppression of aging by the test diet is suggested.

Enhancement of expressions of anti-aging genes was also observed. Anti-aging action of the test diet is suggested.

Muscle fatigue recovery effect of carnosine is known, and it has been considered that the recovery of fatigue is an effect of neutralization of muscular pH. However, since enhancement of genes of mitochondrial system was observed with ingestion of the test diet in the above tests, a novel function thereof exerted for muscles of enhancing mitochondrial functions via the glycolysis system is also suggested. It was also clarified by the above tests that expression of SIRT6 known as a long-life gene was enhanced. It is known that if this gene is overexpressed in a mouse individual, life of the mouse is prolonged. Therefore, it may also be expected that prolongation of life can be provided by use of an imidazole dipeptide. Further, concerning change of expressions of various SLC genes provided by ingestion of the imidazole peptide, a so-called food combination effect may also be expected, that is, it may be expected that if it is eaten together with carnosine, responses of them to various physiologically active substances and food ingredients change.

### 5. Change of serum cytokine concentration

Biochemical test, blood cell count test, blood sugar test, and coagulation test were conducted for blood samples obtained from the test subjects before, during (six weeks after the start of ingestion), and immediately after the food ingestion period of three months. When the test diet was given, a blood sugar level-decreasing tendency was observed. The other indexes were not changed before and after the ingestion, and therefore safety of ingestion of the test diet and placebo diet was reconfirmed.

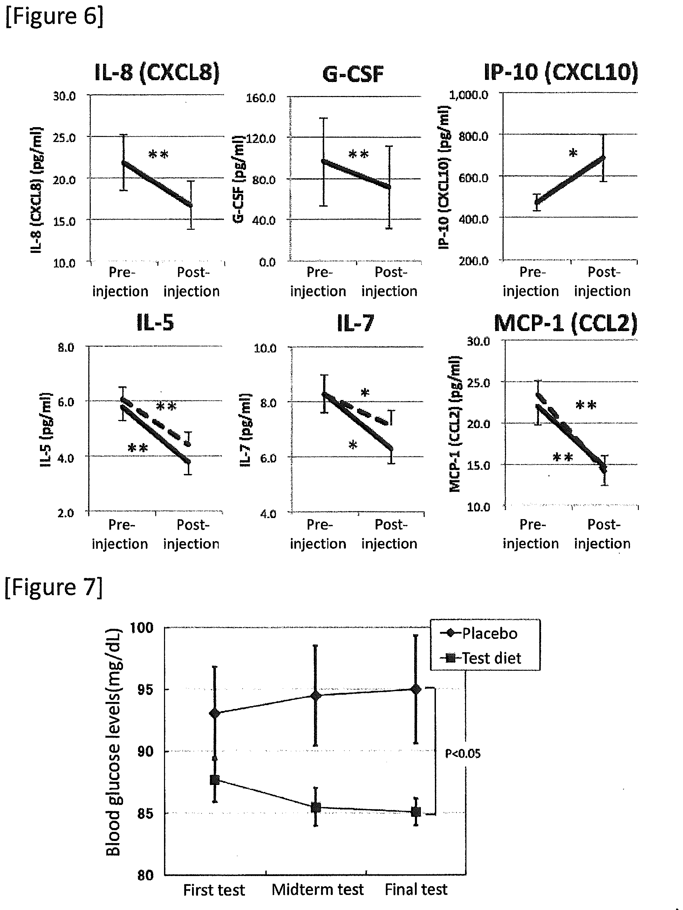

Quantitative analysis of 27 kinds of cytokines and chemokines was carried out for the same blood samples. Quantitative analysis of cytokine concentrations in sera of peripheral bloods of test subjects was conducted by the bead-based multiplex analysis using xMAP technology (Luminex). In this method, cytokines are simultaneously quantitatively analyzed by using specific antibodies bound to beads labeled with different fluorescent substances according to the principle of flow cytometry. The outline of the analysis method using the Bio-Plex Pro™ Human Cytokine Grp I Panel 27-pLex kit (Bio-Rad) is explained below. Antibody beads of different types were put into wells of a 96-well assay plate, and washed twice with the Bio-Plex Wash buffer. Then, serum and a standard solution were added to each well, and the plate was incubated on a shaker at room temperature for 1 hour with shielding light. The beads were washed 3 times with the Wash buffer, then detection antibody solutions were added, and the plate was incubated on a shaker at room temperature for 30 minutes with shielding light. The beads were washed 3 times with the Wash buffer, then a PE-labeled streptavidin solution was added, and the plate was incubated on a shaker at room temperature for 10 minutes with shielding light. The beads were washed 3 times with the Wash buffer, then the Assay buffer was added, and the plate was shaken for 10 seconds with shielding light. PE fluorescence intensity of each type of beads was measured by using Bio-Plex 200 System (Bio-Rad), and concentration of each cytokine in the serum was obtained by using a standard curve created with samples of known amounts. Statistical analysis was performed by paired t-test for the data obtained before and after the ingestion for every test subject, and cytokines for which the test diet group showed difference are shown in Fig. 6.

It was found that blood level of many kinds of cytokine and chemokine molecules including IL-8 (CXCL8) as well as IL-5, IL-7, granulocyte colony-stimulating factor (G-CSF), MCP-1 (CCL2) etc. were significantly reduced by the ingestion of the test diet. It was also found that, on the other hand, the blood level of IP-10 (CXCL10) was significantly increased by the ingestion of the test diet. The placebo diet used in this test contained histidine so that it contained the same amount of essential amino acids as that of the test diet, and it is known that histidine has an anti-inflammatory activity. Therefore, among the aforementioned molecules, blood concentrations of IL-5, IL-7, and MCP-1 (CCL2) were also significantly reduced by the ingestion of the placebo diet.

The blood sugar levels were measured. The results are shown in Fig. 7. The placebo group showed an increasing tendency, and the test diet group showed a decreasing tendency. Thus, the test diet group showed a blood sugar level-improving tendency compared with the placebo group. Since this pilot test was conducted with healthy middle and old age people as the subjects, concentrations of HbA1c (saccharified hemoglobin), which is used as a marker molecule of diabetes, were within a normal range in most of the test subjects, and the concentrations were not changed by the ingestion of the test diet.

### [Evaluation with pathological mice]

High fat diet (HFD) was given to transgenic mice (Alzheimer disease model mice) to induce cerebral hypofunction. Carnosine (L-histidine-β-alanine) was administered to the mice, and influence of carnosine was evaluated.

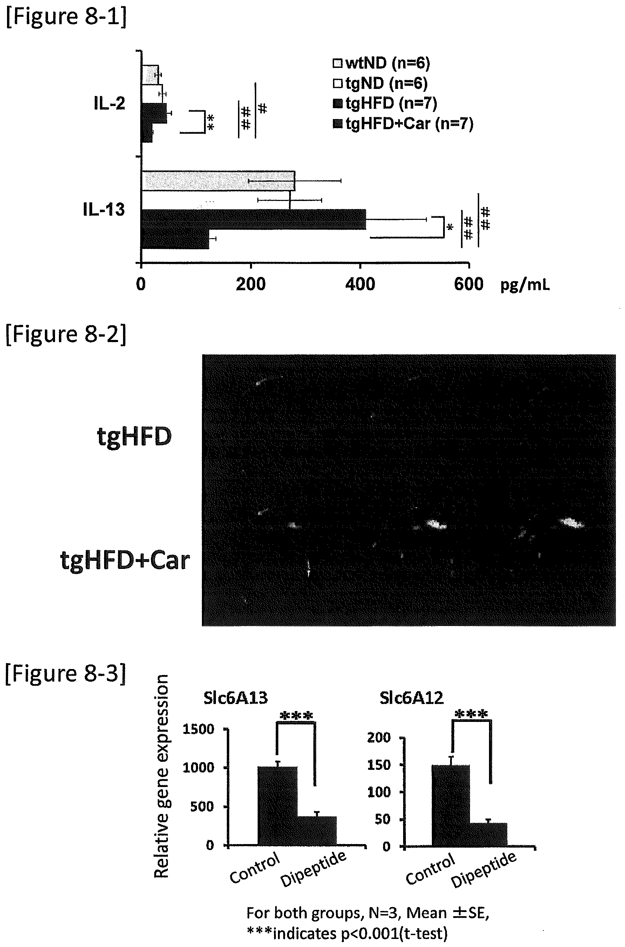

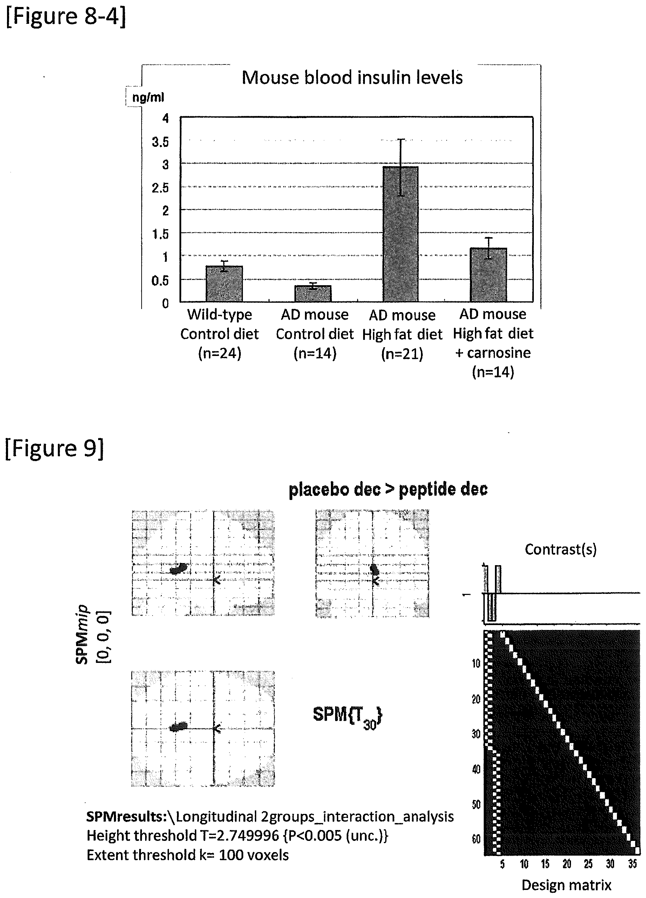

The results are shown in Figs. 8-1, 8-2, 8-3, and 8-4. In the carnosine-containing diet group, cytokines decreased, and suppression of inflammation was suggested (Fig. 8-1). Suppression of cerebral inflammation in the carnosine administration group was also shown by the results of MRI test (Fig. 8-2, red portions). The results of the microarray analysis indicated increase of expressions of GABA transporters such as 7Slc6a12 and slc6a13 observed in the Alzheimer model was suppressed in the carnosine administration group (Fig. 8-3). The amount of GABA that can act as a transmitter is decreased by increase of expressions of the transporters in the Alzheimer disease model mice, and there was suggested a possibility that carnosine suppresses such decrease of the GABA amount.

Blood was collected from mice starved overnight, and blood insulin concentration was determined with a kit (Morinaga). As a result of the blood test, it was found that increase of the blood insulin concentration observed for the high fat diet-induced Alzheimer disease was suppressed in the carnosine administration group (Fig. 8-4).

### [Evaluation 2 with healthy volunteers]

The same test diet containing chicken-derived imidazole dipeptides as mentioned above was given to test subjects (healthy volunteers 40 years old or older, divided into test diet group and placebo diet group) over three months, and before, during, and after the ingestion period, change of cerebral functions etc. were evaluated. Compositions of the test diet and placebo diet (as daily amounts) are as shown in Table 1. A first pilot test and second pilot test were conducted.

The total numbers of the test subjects subjected to the first and second test were 30 subjects for the test diet group and 30 subjects for the control diet group (placebo diet group) (refer to the following tables).

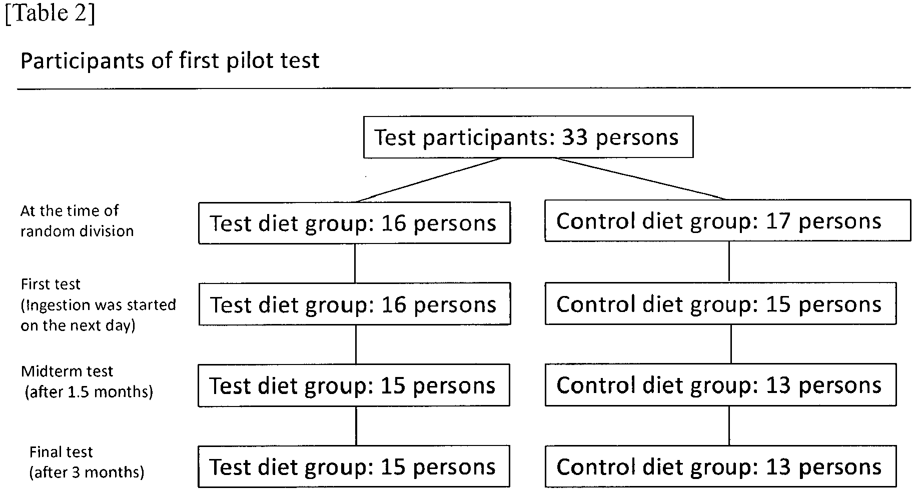

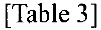

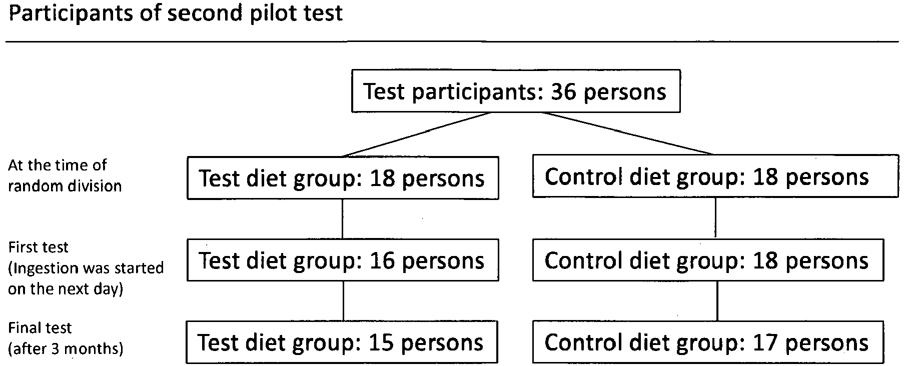

### 1. Analysis by MRI imaging

In the second pilot test, MRI imaging test was performed to directly measure the cerebral blood flow, which changes with advance of dementia.

Change of cerebral blood flow can be measured by the arterial spin labeling method, which is a method of measuring blood flow change by using an MRI apparatus using magnetism without using a labeling compound, or the like.

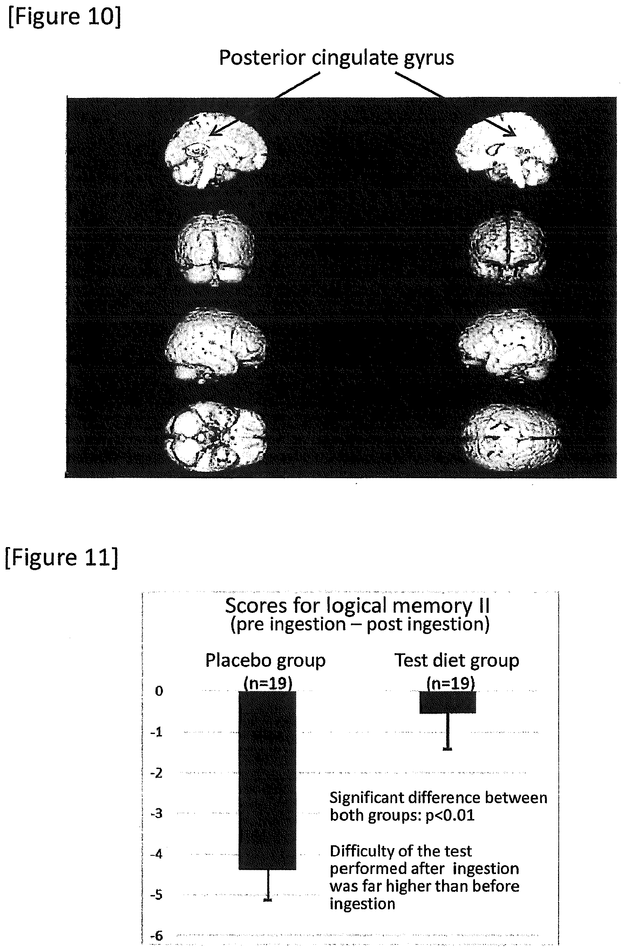

The results are shown in Figs. 9 and 10. It was found that blood flow in the posterior cingulated gyrus, where blood flow changes with the advance and onset from a predemential stage, was maintained in the test diet ingestion group with a significant difference (p < 0.005) compared with the placebo diet group.

### 2. Subgroup analysis

For the subgroups of the test subjects (60 years old or older) who participated in the first and second pilot tests, logical memory, which degrades with advance and onset from a predemential stage, was evaluated (delayed word recall task).

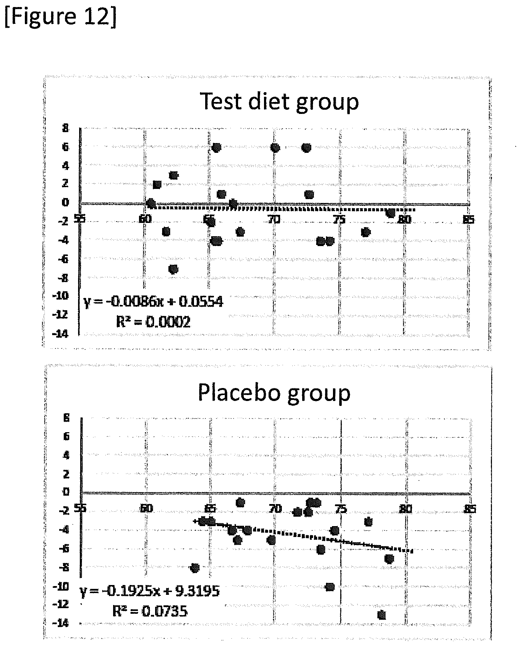

The results are shown in Figs. 11 and 12. In this test, difficulty of the second test was higher than that of the first test, and therefore the scores tended to worsen in the second test compared with first test. However, it was found that the degradation of the scores was suppressed in the test diet ingestion group compared with the placebo diet group with a strong statistical significance (p < 0.01).

The results of these two experiments suggest that the agent containing the imidazole dipeptides derived from chicken has an action of preventing aging of cerebral functions and onset of dementia.

### [Preparation Examples]

### (1) Preparation of chicken extract

The test diet containing chicken-derived imidazole dipeptides was prepared according to the following process.

Chicken breast meat was minced with a meat grinder, and warm water was added to the chicken breast meat in a weight of 1.5 times the weight of the meat. The mixture was heated at 90°C for 4 hours, thereby concentrated until Brix became 20% or higher, and subjected to diatomaceous earth filtration and ultrafiltration so that it finally had a carnosine + anserine concentration of about 10% (w/v %).

### (2) Capsules

Carnosine (1.0 weight part), placental extract (powder, 0.2 weight part), and lactose (1.3 weight parts were mixed into a uniform mixture, and filled into hard capsules in a conventional manner to prepare 250 mg net weight capsules (100 mg of carnosine/capsule).

### (3) Tablets

There were prepared tablets containing 60 mg of a mixture of carnosine and anserine per one tablet (300 mg) together with maltose, dextrin, starch, vitamin E-containing vegetable oil, isomaltooligosaccharides, hardly digestible dextrin, shellfish calcium, trehalose, sucrose ester, vitamin C, citric acid, calcium phosphate, perfume, shellac, niacin, vitamin K, sweetener, potassium chloride, vitamin A, calcium pantothenate, biotin, iron pyrophosphate, B vitamins, vitamin D, magnesium carbonate, and folic acid.

### Sequence Listing Free Text

SEQ ID NO: 1 - SLC23A2, NM_203327
SEQ ID NO: 2 - SLC43A2, NM_152346
SEQ ID NO: 3 -- SLC29A3, NM 018344
SEQ ID NO: 4 -- SLC35C1, NM_018389
SEQ ID NO: 5 -- SLC25A33, NM _032315
SEQ ID NO: 6 -- SLC22A23, NM_015482
SEQ ID NO: 7 - CXCL12, NM_199168
SEQ ID NO: 8 -- CCL17, NM_002987
SEQ ID NO: 9 -- TSPO, NM_000714
SEQ ID NO: 10 - P2RY1, NM_002563
SEQ ID NO: 11 -- CAMK1, NM_003656
SEQ ID NO: 12 -- ACO2, NM_001098
SEQ ID NO: 13 -- ATP7A, NM_000052
SEQ ID NO: 14 -- POLG, NM_002693
SEQ ID NO: 15 -- IDH3G, NM_004135
SEQ ID NO: 16 -- UCP2, NM 003355
SEQ ID NO: 17 -- BCKDHA, NM_000709
SEQ ID NO: 18 -- TAP2, NM_018833
SEQ ID NO: 19 -- SMARCD1, NM_139071
SEQ ID NO: 20 -- SIRT6 and NM_016539

## Claims

1. An agent for use in preventing onset of dementia due to aging, which contains at least one imidazole dipeptide.

2. An agent for use in suppressing a cerebral atrophy, which contains at least one imidazole dipeptide.

3. The agent for use according to claim 1 or 2, wherein the agent is for ingestion of 200 mg or more as a daily dose of the at least one imidazole dipeptide.

4. The agent for use according to any one of claims 1 to 3, wherein at least one kind of the imidazole dipeptide is derived from chicken.

5. The agent for use according to any one of claims 1 to 4, which further contains creatine and a nucleic acid.

6. The agent for use according to any one of claims 1 to 5, which is for an elderly person or a person with a slight mood disorder.

## Patentansprüche

1. Agens zur Verwendung in der Prävention des Beginns von altersbedingter Demenz, das mindestens ein Imidazol-Dipeptid enthält.

2. Agens zur Verwendung in der Suppression zerebraler Atrophie, das mindestens ein Imidazol-Dipeptid enthält.

3. Agens zur Verwendung nach Anspruch 1 oder 2, wobei das Agens zur Einnahme von 200 mg oder mehr als tägliche Dosis von dem mindestens einen Imidazol-Dipeptid ist.

4. Agens zur Verwendung nach einem der Ansprüche 1 bis 3, wobei mindestens eine Art des Imidazol-Dipeptids aus dem Huhn abgeleitet ist.

5. Agens zur Verwendung nach einem der Ansprüche 1 bis 4, das weiterhin Kreatin und eine Nukleinsäure enthält.

6. Agens zur Verwendung nach einem der Ansprüche 1 bis 5, das für eine ältere Person oder eine Person mit einer leichten Stimmungsschwankungserkrankung ist.

## Revendications

1. Agent pour son utilisation dans la prévention de l'apparition d'une démence due au vieillissement, qui contient au moins un dipeptide d'imidazole.

2. Agent pour son utilisation dans la suppression d'une atrophie cérébrale, qui contient au moins un dipeptide d'imidazole.

3. Agent pour son utilisation selon la revendication 1 ou 2, dans lequel l'agent est destiné à être ingéré à raison de 200 mg ou plus en tant que dose quotidienne au moins dudit dipeptide d'imidazole.

4. Agent pour son utilisation selon l'une quelconque des revendications 1 à 3, dans lequel au moins un type du dipeptide d'imidazole est dérivé du poulet.

5. Agent pour son utilisation selon l'une quelconque des revendications 1 à 4, qui contient en outre de la créatine et un acide nucléique.

6. Agent pour son utilisation selon l'une quelconque des revendications 1 à 5, qui est destiné à une personne âgée ou à une personne souffrant d'un léger trouble de l'humeur.
